(19) Europäisches Patentamt
European Patent Office
Office européen des brevets

(11) EP 4 656 679 A1

(12) **EUROPEAN PATENT APPLICATION**
published in accordance with Art. 153(4) EPC

(43) Date of publication:
**03.12.2025 Bulletin 2025/49**

(21) Application number: **24760240.2**

(22) Date of filing: **15.02.2024**

(51) International Patent Classification (IPC):
***C08L 11/02*** (2006.01) ***A41D 19/00*** (2006.01)
***C08K 3/06*** (2006.01) ***C08K 3/22*** (2006.01)
***C08K 5/378*** (2006.01)

(52) Cooperative Patent Classification (CPC):
**A41D 19/00; C08K 3/06; C08K 3/22; C08K 5/378; C08L 11/02**

(86) International application number:
**PCT/JP2024/005234**

(87) International publication number:
**WO 2024/176939 (29.08.2024 Gazette 2024/35)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **20.02.2023 JP 2023024179**

(71) Applicant: **Denka Company Limited**
**Tokyo 103-8338 (JP)**

(72) Inventors:
- **UENO, Yumemi**
  **Tokyo 103-8338 (JP)**
- **KUMAGAI, Yushi**
  **Tokyo 103-8338 (JP)**

(74) Representative: **Gulde & Partner**
**Patent- und Rechtsanwaltskanzlei mbB**
**Berliner Freiheit 2**
**10785 Berlin (DE)**

# (54) CHLOROPRENE-BASED POLYMER LATEX COMPOSITION AND DIP-MOLDED BODY

(57) To provide a chloroprene-based polymer latex composition capable of providing a dip-molded body excellent in flexibility and excellent in tensile strength at break.

According to the present invention, there is provided a chloroprene-based polymer latex composition comprising: a chloroprene-based polymer latex (A), a metal oxide (B), a vulcanization accelerator (C), sulfur (D), and a heteroaromatic ring compound (E), wherein: a toluene-insoluble content of a chloroprene-based polymer rubber obtained by freeze-drying the chloroprene-based polymer latex (A) is 50 to 95% by mass; the chloroprene-based polymer latex composition comprises 0.3 to 15.0 parts by mass of the metal oxide (B), 0.02 to 1.50 parts by mass of the vulcanization accelerator (C), 0.01 to 0.75 parts by mass of the sulfur (D), and 0.1 to 10.0 parts by mass of the heteroaromatic ring compound (E), with respect to 100 parts by mass of a solid content of the chloroprene-based polymer latex (A); the vulcanization accelerator (C) comprises at least one selected from a group consisting of a thiuram-based vulcanization accelerator, a dithiocarbamate-based vulcanization accelerator, a thiourea-based vulcanization accelerator, a guanidine-based vulcanization accelerator, a xanthate-based vulcanization accelerator, and a thiazole-based vulcanization accelerator; and the heteroaromatic ring compound (E) is represented by general formula (1).

EP 4 656 679 A1

## Description

TECHNICAL FIELD

The present invention relates to a chloroprene-based polymer latex composition, and a dip-molded body.

BACKGROUND ART

Chloroprene-based polymers are known as materials for dip-molded products such as medical surgical gloves, medical examination gloves, industrial gloves, balloons, catheters, and rubber boots.

Various technologies related to chloroprene-based polymers for dip-molded products have been proposed. Patent Literature 1 describes that for anti-vibration rubber applications, mixing a low molecular weight chloroprene polymer having a number average molecular weight in the range of 500 to 50,000 improves damping performance. Patent Literature 2 describes, for dip-molded product applications, a polychloroprene latex with a pH of 7 to 14, containing 100 parts by mass of a modified polychloroprene obtained by copolymerizing chloroprene and methacrylic acid, 90 to 150 parts by mass of water, 1 to 5 parts by mass of an emulsifier, and 0.5 to 2.5 parts by mass of potassium ions. Patent Literature 3 describes, for dip-molded product applications, a mercaptan-modified polychloroprene latex obtained by copolymerizing chloroprene and 2,3-dichloro-1,3-butadiene, wherein in the 13C-solid NMR spectrum of the polychloroprene, a peak area (A) at 126.2 to 127.6 ppm, a peak area (B) at 122.0 to 126.2 ppm, and a peak area (C) at 129.9 to 130.3 ppm are within the range represented by the following general formula (I). Patent Literature 4 describes, for dip-molded product applications, a chloroprene polymer latex that can achieve both excellent flexibility and mechanical properties in a vulcanized rubber produced by dip-molding, by including a high molecular weight component and a low molecular weight component.

$$\frac{4.0}{100} \leqq \frac{A}{B-C} \leqq \frac{5.8}{100} \quad \cdots \quad (\mathrm{I})$$

CITATION LIST

PATENT LITERATURE

[Patent Literature 1] JP-A-Hei 7-292165 A
[Patent Literature 2] JP-A-2014-114342 A
[Patent Literature 3] WO 2019/009038
[Patent Literature 4] JP-A-2019-143002 A

SUMMARY OF INVENTION

TECHNICAL PROBLEM

In dip-molded bodies using a chloroprene-based polymer, high flexibility similar to that of dip-molded bodies obtained using natural rubber or polyisoprene is required, and furthermore, there is a tendency for higher mechanical properties to be demanded. Improvement in flexibility is also related to improvements in wearing comfort and the texture of a film. Therefore, a chloroprene-based polymer latex composition capable of providing a dip-molded body excellent in flexibility and also excellent in tensile strength at break has been desired.

The present invention has been made in view of such circumstances, and an object thereof is to provide a chloroprene-based polymer latex composition capable of providing a dip-molded body excellent in flexibility and also excellent in tensile strength at break, which has been difficult with conventional chloroprene-based polymer latex compositions.

Hereinafter, various embodiments of the present invention will be exemplified. The embodiments shown below can be combined with each other.

[1] A chloroprene-based polymer latex composition comprising:

a chloroprene-based polymer latex (A),

a metal oxide (B),
a vulcanization accelerator (C),
sulfur (D), and
a heteroaromatic ring compound (E), wherein:

a toluene-insoluble content of a chloroprene-based polymer rubber obtained by freeze-drying the chloroprene-based polymer latex (A) is 50 to 95% by mass;
the chloroprene-based polymer latex composition comprises 0.3 to 15.0 parts by mass of the metal oxide (B), 0.02 to 1.50 parts by mass of the vulcanization accelerator (C), 0.01 to 0.75 parts by mass of the sulfur (D), and
0.1 to 10.0 parts by mass of the heteroaromatic ring compound (E), with respect to 100 parts by mass of a solid content of the chloroprene-based polymer latex (A);
the vulcanization accelerator (C) comprises at least one selected from a group consisting of a thiuram-based vulcanization accelerator, a dithiocarbamate-based vulcanization accelerator, a thiourea-based vulcanization accelerator, a guanidine-based vulcanization accelerator, a xanthate-based vulcanization accelerator, and a thiazole-based vulcanization accelerator; and
the heteroaromatic ring compound (E) is represented by general formula (1),

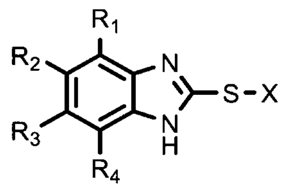

(1)

wherein, in the general formula (1), X represents a hydrogen atom or a metal atom, and $R_1$ to $R_4$ each independently represent a hydrogen atom, an optionally substituted alkyl group, an optionally substituted ether group, a nitro group, an amino group, or a carboxyl group, and $R_1$ to $R_4$ may be the same or different from each other.

[2] The chloroprene-based polymer latex composition of [1], comprising 0.5 to 10.0 parts by mass of an antioxidant with respect to 100 parts by mass of the solid content of the chloroprene-based polymer latex (A).
[3] The chloroprene-based polymer latex composition of [1] or [2], wherein a molecular weight distribution obtained by gel permeation chromatography measurement of a tetrahydrofuran-soluble content in the chloroprene-based polymer latex (A) has a peak with a weight average molecular weight of 5,000 to 80,000.
[4] The chloroprene-based polymer latex composition of any one of [1] to [3], wherein a ratio RB/RA is 0.10 or more, wherein the ratio RB/RA is a ratio of a total peak area RB of abietic acid, neoabietic acid, palustric acid, levopimaric acid, and salts thereof to a total peak area RA of dehydroabietic acid, pimaric acid, isopimaric acid, dihydroabietic acid, and salts thereof, obtained from a gas chromatography analysis of an extract extracted with an ethanol and toluene azeotropic mixture defined by JIS K 6229 from a dip-molded body obtained by molding the chloroprene-based polymer latex composition by a dip coagulation method and heat-drying at 140°C for 60 minutes.
[5] The chloroprene-based polymer latex composition of any one of [1] to [4], wherein a dip-molded body, obtained by molding the chloroprene-based polymer latex composition by a dip coagulation method and heat-drying at 140°C for 60 minutes, has a 100% elongation modulus of 0.65 MPa or less, measured based on JIS K 6251, and a tensile strength at break of 19.0 MPa or more, measured based on JIS K 6251.
[6] A dip-molded body of the chloroprene-based polymer latex composition of any one of [1] to [5].
[7] The dip-molded body of [6], wherein the dip-molded body is a glove for industrial or general household use, a medical glove, a balloon, a catheter, or a boot.

## EFFECTS OF INVENTION

According to the chloroprene-based polymer latex composition of the present invention, a dip-molded body excellent in flexibility and also excellent in tensile strength at break can be obtained. Furthermore, the obtained dip-molded body can be used as various materials requiring flexibility, tensile strength at break, and the like, by leveraging these properties. Specifically, it can be used as industrial gloves, general household gloves, medical gloves, balloons, catheters, or boots.

DESCRIPTION OF EMBODIMENTS

Hereinafter, the present invention will be described in detail by exemplifying embodiments of the present invention. The present invention is not limited in any way by these descriptions. The features of the embodiments of the present invention shown below can be combined with each other. In addition, an invention can be independently established for each feature.

1. Chloroprene-based Polymer Latex Composition

The chloroprene-based polymer latex composition according to the present invention comprises a chloroprene-based polymer latex (A), a metal oxide (B), a vulcanization accelerator (C), sulfur (D), and a heteroaromatic ring compound (E). Hereinafter, components that can be included in the chloroprene-based polymer latex composition according to the present invention will be described.

1.1 Chloroprene-based Polymer Latex (A)

The chloroprene-based polymer latex (A) according to the present invention comprises a chloroprene-based polymer. The chloroprene-based polymer according to the present invention means a polymer comprising a monomer unit derived from 2-chloro-1,3-butadiene (hereinafter, also referred to as chloroprene).

<Monomer Unit that Chloroprene-based Polymer May Comprise>

A chloroprene-based polymer according to one embodiment of the present invention can also be a copolymer of chloroprene and another monomer copolymerizable with chloroprene, and examples of the other monomer include 1-chloro-1,3-butadiene, 2,3-dichloro-1,3-butadiene, isoprene, styrene, methacrylic acid, acrylonitrile, sulfur, and the like, and as the other monomer, one of these may be used, or two or more thereof may be used in combination.

A chloroprene-based polymer according to one embodiment of the present invention preferably comprises a chloroprene monomer unit and 2,3-dichloro-1,3-butadiene monomer unit. The chloroprene-based polymer according to one embodiment of the present invention preferably comprises 0 to 30% by mass, more preferably 8 to 20% by mass, and still more preferably 10 to 17% by mass of 2,3-dichloro-1,3-butadiene monomer units, with respect to 100% by mass of the total of chloroprene monomer units and 2,3-dichloro-1,3-butadiene monomer units. The content rate of 2,3-dichloro-1,3-butadiene monomer units is, for example, 0, 1, 2, 3, 4, 5, 6, 7, 8, 9, 10, 11, 12, 13, 14, 15, 16, 17, 18, 19, 20, 21, 22, 23, 24, 25, 26, 27, 28, 29, or 30% by mass, and may be within a range between any two of the numerical values exemplified here.

A chloroprene-based polymer latex (A) according to one embodiment of the present invention can comprise 70 to 100% by mass of chloroprene monomer units, with respect to 100% by mass of the chloroprene-based polymer comprised in the chloroprene-based polymer latex (A). The content of chloroprene monomer units is, for example, 70, 75, 80, 85, 90, 91, 92, 93, 94, 95, 96, 97, 98, 99, or 100% by mass, and may be within a range between any two of the numerical values exemplified here.

A chloroprene-based polymer latex (A) according to one embodiment of the present invention can comprise 0 to 30% by mass of other monomer units other than chloroprene monomer units and 2,3-dichloro-1,3-butadiene monomer units, when the chloroprene-based polymer comprised in the chloroprene-based polymer latex (A) is taken as 100% by mass. The content rate of other monomer units is, for example, 0, 1, 2, 3, 4, 5, 6, 7, 8, 9, 10, 11, 12, 13, 14, 15, 16, 17, 18, 19, 20, 21, 22, 23, 24, 25, 26, 27, 28, 29, or 30% by mass, and may be within a range between any two of the numerical values exemplified here. The chloroprene-based polymer according to one embodiment of the present invention may consist of chloroprene monomer units and 2,3-dichloro-1,3-butadiene monomer units.

When the chloroprene-based polymer latex (A) according to one embodiment of the present invention comprises two or more different chloroprene-based polymers, or is a mixture of two or more different chloroprene-based polymer latexes, the content of each monomer unit means the total of each monomer unit in all chloroprene-based polymers comprised in the chloroprene-based polymer latex (A).

<Toluene-insoluble Content>

The chloroprene-based polymer latex (A) according to the present invention has a toluene-insoluble content of 50 to 95% by mass. The chloroprene-based polymer latex (A) according to one embodiment of the present invention more preferably has a toluene-insoluble content of 55 to 85% by mass. The toluene-insoluble content is, for example, 50, 55, 60, 65, 70, 75, 80, 85, 90, or 95% by mass, and may be within a range between any two of the numerical values exemplified here.

The toluene-insoluble content can be determined by the following formula, when a chloroprene-based polymer

rubber obtained by freeze-drying a chloroprene-based polymer latex is immersed in toluene for 16 hours, the chloroprene-based polymer rubber after freeze-drying is A g, and a gel fraction (insoluble fraction) separated from a mixture dissolved in toluene is B g. The toluene-insoluble content, specifically, can be determined by the method described in Examples.

$$\text{Toluene-insoluble content (gel fraction)} = B/A \times 100\ (\%)$$

The toluene-insoluble content can be controlled by adjusting production conditions of the chloroprene-based polymer rubber, for example, a polymerization formulation (formulation such as type and amount of a chain transfer agent, polymerization temperature, polymerization time, polymerization conversion rate, and the like) of polymerization comprised in the chloroprene-based polymer latex (A), and by adjusting the type and amount of a polymer blend.

<Weight Average Molecular Weight>

A chloroprene-based polymer latex (A) according to one embodiment of the present invention can have a peak with a weight average molecular weight of 200,000 to 1,500,000 in a molecular weight distribution obtained by gel permeation chromatography measurement of a tetrahydrofuran-soluble content in the chloroprene-based polymer latex (A). Moreover, the chloroprene-based polymer latex (A) according to one embodiment of the present invention preferably has a peak with a weight average molecular weight of 5,000 to 80,000 in a molecular weight distribution obtained by gel permeation chromatography measurement of a tetrahydrofuran-soluble content in the chloroprene-based polymer latex (A). It is preferable that in a molecular weight distribution obtained when a sample, prepared by mixing the chloroprene-based polymer latex (A) according to one embodiment of the present invention with a large amount of methanol, precipitating, filtering, and drying to obtain a chloroprene-based polymer, and dissolving the chloroprene-based polymer in tetrahydrofuran, is measured by gel permeation chromatography, a peak with a weight average molecular weight of 200,000 to 1,500,000 and/or a peak with a weight average molecular weight of 5,000 to 80,000 is detected.

Moreover, in a chloroprene-based polymer latex (A) according to one embodiment of the present invention, it is preferable that in a molecular weight distribution obtained when a sample, prepared by mixing the chloroprene-based polymer latex (A) with a large amount of methanol, precipitating, filtering, and drying to obtain a chloroprene-based polymer, and dissolving the chloroprene-based polymer in tetrahydrofuran, is measured by gel permeation chromatography, a peak with a weight average molecular weight of 200,000 to 1,500,000 is detected, and furthermore, a peak with a weight average molecular weight of 5,000 to 80,000 is detected. Also, in the molecular weight distribution, it is preferable that a peak with a weight average molecular weight of 300,000 to 1,500,000 is detected, and more preferably, a peak with a weight average molecular weight of 5,000 to 50,000 is further detected. Also, in the molecular weight distribution, it is preferable that a peak with a weight average molecular weight of 500,000 to 1,500,000 is detected, and still more preferably, a peak with a weight average molecular weight of 8,000 to 30,000 is detected.

A chloroprene-based polymer latex (A) according to one embodiment of the present invention can comprise two or more types of polymers with different weight average molecular weights, a chloroprene-based polymer $\alpha$ and a chloroprene-based polymer $\beta$. Monomer units that the chloroprene-based polymer $\alpha$ and the chloroprene-based polymer $\beta$ may comprise and their contents are as described above for monomer units that a chloroprene-based polymer may comprise. The chloroprene-based polymer $\alpha$ according to one embodiment of the present invention can have a different weight average molecular weight from the chloroprene-based polymer $\beta$. The difference between the weight average molecular weight of the chloroprene-based polymer $\alpha$ and the weight average molecular weight of the chloroprene-based polymer $\beta$ according to one embodiment of the present invention is preferably 100,000 or more, and more preferably 400,000 or more. The difference between the weight average molecular weight of the chloroprene-based polymer $\alpha$ and the weight average molecular weight of the chloroprene-based polymer $\beta$ described later is, for example, 100,000, 200,000, 300,000, 400,000, 500,000, 600,000, 700,000, 800,000, 900,000, 1,000,000, 1,200,000, 1,300,000, 1,400,000, or 1,500,000, and may be within a range between any two of the numerical values exemplified here.

As one example, the weight average molecular weight of the chloroprene-based polymer $\alpha$ can be smaller than the molecular weight of the chloroprene-based polymer $\beta$. The weight average molecular weight of the chloroprene-based polymer $\alpha$ can be 5,000 to 80,000. The weight average molecular weight of the chloroprene-based polymer $\alpha$ is, for example, 5,000, 6,000, 7,000, 8,000, 9,000, 10,000, 11,000, 12,000, 13,000, 14,000, 15,000, 16,000, 17,000, 18,000, 19,000, 20,000, 21,000, 22,000, 23,000, 24,000, 25,000, 26,000, 27,000, 28,000, 29,000, 30,000, 35,000, 40,000, 50,000, 60,000, 70,000, or 80,000, and may be within a range between any two of the numerical values exemplified here.

As one example, the weight average molecular weight of the chloroprene-based polymer $\beta$ can be larger than the molecular weight of the chloroprene-based polymer $\alpha$. The weight average molecular weight of the chloroprene-based polymer $\beta$ can be 200,000 to 1,500,000. The weight average molecular weight of the chloroprene-based polymer $\beta$ is, for example, 200,000, 300,000, 400,000, 500,000, 600,000, 700,000, 800,000, 900,000, 1,000,000, 1,100,000, 1,200,000, 1,300,000, 1,400,000, or 1,500,000, and may be within a range between any two of the numerical values exemplified here.

A chloroprene-based polymer latex (A) according to one embodiment of the present invention preferably comprises 5 to 40% by mass of the chloroprene-based polymer $\alpha$, when the polymer comprised in the chloroprene-based polymer latex (A) is taken as 100% by mass. The content rate of the chloroprene-based polymer $\alpha$ can be, for example, 5, 10, 15, 20, 25, 30, 35, or 40% by mass, and may be within a range between any two of the numerical values exemplified here.

A chloroprene-based polymer latex (A) according to one embodiment of the present invention preferably comprises 60 to 95% by mass of the chloroprene-based polymer $\beta$, when the polymer comprised in the chloroprene-based polymer latex (A) is taken as 100% by mass. The content rate of the chloroprene-based polymer $\beta$ is, for example, 60, 65, 70, 75, 80, 85, 90, or 95% by mass, and may be within a range between any two of the numerical values exemplified here.

The weight average molecular weight can be determined, for example, by analyzing a sample, prepared by mixing the chloroprene-based polymer latex (A) with a large amount of methanol, precipitating, filtering, and drying to obtain a chloroprene-based polymer, and dissolving the chloroprene-based polymer in tetrahydrofuran, using gel permeation chromatography (GPC). The GPC measurement conditions can be as described in Examples. The weight average molecular weight can be controlled by adjusting production conditions, specifically, a polymerization formulation of the chloroprene-based polymer (formulation such as type and amount of a chain transfer agent, polymerization temperature, polymerization time, polymerization conversion rate, and the like).

1.2 Method for Producing Chloroprene-based Polymer Latex (A)

The method for producing the chloroprene-based polymer latex (A) according to the present invention is not particularly limited, but it can be obtained, for example, by the following method.

A method for producing the chloroprene-based polymer latex (A) according to a first embodiment of the present invention can include a polymerization step of polymerizing a raw material monomer comprising chloroprene to obtain a chloroprene-based polymer latex, and the obtained chloroprene-based polymer latex can be used as the chloroprene-based polymer latex (A) as is.

A method for producing the chloroprene-based polymer latex (A) according to a second embodiment of the present invention can include a polymerization step of polymerizing a raw material monomer comprising chloroprene to obtain a chloroprene-based polymer latex, and a mixing step of mixing two or more types of latexes to obtain the chloroprene-based polymer latex (A).

A method for producing the chloroprene-based polymer latex (A) according to a third embodiment of the present invention can include:

a first polymerization step of polymerizing a raw material monomer comprising chloroprene to polymerize a chloroprene-based polymer $\alpha$, and a second polymerization step of polymerizing a chloroprene-based polymer $\beta$ in the presence of the chloroprene-based polymer $\alpha$, or,

a first polymerization step of polymerizing a raw material monomer comprising chloroprene to polymerize a chloroprene-based polymer $\beta$, and a second polymerization step of polymerizing a chloroprene-based polymer $\alpha$ in the presence of the chloroprene-based polymer $\beta$.

Hereinafter, a method for producing the chloroprene-based polymer latex (A) according to the first embodiment of the present invention will be described. The method for producing the chloroprene-based polymer latex (A) according to one embodiment of the present invention can comprise a polymerization step of polymerizing a raw material monomer comprising chloroprene to obtain a chloroprene-based polymer latex.

In the polymerization step, the raw material monomer comprises chloroprene, and can also comprise another monomer copolymerizable with chloroprene. The other monomer copolymerizable with chloroprene is as described above. The type and charged amount of each monomer are preferably adjusted so that the type and content of each monomer unit in the obtained polymer are within the numerical ranges described above. As one example, the copolymerization amount of 2,3-dichloro-1,3-butadiene in the chloroprene-based polymer comprised in the chloroprene-based polymer latex can be in the range of 0 to 30% by mass with respect to 100% by mass of the total of chloroprene monomer and 2,3-dichloro-1,3-butadiene in the chloroprene-based polymer, and in this case, it is preferable that the charged amount of 2,3-dichloro-1,3-butadiene before the start of emulsion polymerization is in the range of 0 to 30 parts by mass with respect to 100 parts by mass of the total of chloroprene monomer and 2,3-dichloro-1,3-butadiene monomer. From the viewpoint of polymerization control, it is more preferable that the charged amount of 2,3-dichloro-1,3-butadiene is 5 to 25 parts by mass with respect to 100 parts by mass of the total of chloroprene monomer and 2,3-dichloro-1,3-butadiene monomer.

When producing a chloroprene-based polymer, the raw material monomer can be polymerized by a polymerization method such as emulsion polymerization, solution polymerization, suspension polymerization, or bulk polymerization. Among these polymerization methods, emulsion polymerization is suitable due to various advantages such as being easy to control, easy to take out the polymer from a polymerization reaction mixture, and having a relatively high

polymerization rate.

Emulsion polymerization is a type of radical polymerization, and is a method of polymerizing by charging a raw material monomer into a reaction vessel together with a chain transfer agent, water, an alkali (for example, a metal hydroxide such as potassium hydroxide or sodium hydroxide), an emulsifier (dispersant), a reducing agent (for example, sodium bisulfite), a polymerization initiator, and the like.

The type of chain transfer agent used during emulsion polymerization is not particularly limited, and for example, long-chain alkyl mercaptans such as n-dodecyl mercaptan and tert-dodecyl mercaptan, dialkyl xanthogen disulfides such as diisopropyl xanthogen disulfide and diethyl xanthogen disulfide, iodoform, and the like, which are known chain transfer agents generally used for emulsion polymerization of chloroprene, can be used. As the chain transfer agent, long-chain alkyl mercaptans are preferable, and n-dodecyl mercaptan is more preferable.

By adjusting the type and amount of the chain transfer agent, the weight average molecular weight of the obtained chloroprene-based polymer latex can be adjusted.

As one example, in order to obtain a chloroprene-based polymer latex comprising a chloroprene-based polymer β having a weight average molecular weight of 200,000 to 1,500,000, it is preferable that the charged amount of the chain transfer agent before the start of emulsion polymerization is 0.01 parts by mass or more with respect to 100 parts by mass of the raw material monomer (for example, 100 parts by mass of the total of chloroprene and 2,3-dichloro-1,3-butadiene). From the viewpoint of obtaining a chloroprene-based polymer latex comprising a chloroprene-based polymer β having a weight average molecular weight of 200,000 to 1,500,000, the charged amount of the chain transfer agent is more preferably 0.02 to 0.05 parts by mass, for example, 0.01, 0.02, 0.03, 0.04, 0.05, 0.06, 0.07, 0.08, 0.09 parts by mass, or less than 0.10 parts by mass, and may be within a range between any two of the numerical values exemplified here. When the charged amount of the chain transfer agent, particularly long-chain alkyl mercaptans, is 0.01 parts by mass or more, the storage stability of the latex is further improved, and when the charged amount is less than 0.10 parts by mass, particularly less than 0.05 parts by mass, the toluene-insoluble content becomes larger, and the tensile strength at break of a dip-molded body comprising the obtained chloroprene-based polymer latex becomes higher.

As another example, in order to obtain a chloroprene-based polymer latex comprising a chloroprene-based polymer α exhibiting a peak with a weight average molecular weight of 5,000 to 80,000 in a molecular weight distribution, it is preferable that the charged amount of the chain transfer agent before the start of emulsion polymerization is 1.0 to 10.0 parts by mass with respect to 100 parts by mass of the monomer. In this case, the charged amount of the chain transfer agent is, for example, 1.0, 2.0, 3.0, 4.0, 5.0, 6.0, 7.0, 8.0, 9.0, or 10.0 parts by mass, and may be within a range between any two of the numerical values exemplified here.

In the method for producing the chloroprene-based polymer latex (A) according to the first embodiment of the present invention, the chloroprene-based polymer latex obtained in the polymerization step can be used as the chloroprene-based polymer latex (A) as is, and in this case, in the polymerization step, it is preferable to obtain a chloroprene-based polymer latex comprising a chloroprene-based polymer β having a weight average molecular weight of 200,000 to 1,500,000.

The emulsifier preferably comprises a rosin acid and/or a rosin acid salt. Examples of the rosin acid salt include alkali metal salts such as a sodium salt and a potassium salt. The addition amount of the rosin acid and the rosin acid salt can be 3.0 to 7.0 parts by mass with respect to 100 parts by mass of the raw material monomer used. The addition amount of the rosin acid and the rosin acid salt is, for example, 3.0, 3.5, 4.0, 4.5, 5.0, 5.5, 6.0, 6.5, or 7.0 parts by mass, and may be within a range between any two of the numerical values exemplified here. By using rosin acids, aggregation of rubber solid content and pH fluctuation can be prevented when blended with a base latex.

The rosin acid and/or the rosin acid salt can comprise a conjugated resin acid component and a non-conjugated resin acid component. Examples of the conjugated resin acid component include abietic acid, neoabietic acid, palustric acid, levopimaric acid, and salts thereof, and the like. Examples of the non-conjugated resin acid component include dehydroabietic acid, pimaric acid, isopimaric acid, dihydropimaric acid, dihydroabietic acid, and salts thereof, and the like.

The rosin acid and rosin acid salt used in the emulsion polymerization step can be such that a ratio RB/RA is 0.10 or more and 0.70 or less, where RA is a total peak area of dehydroabietic acid, pimaric acid, isopimaric acid, dihydroabietic acid, and salts thereof, and RB is a total peak area of abietic acid, neoabietic acid, palustric acid, levopimaric acid, and salts thereof, obtained from a gas chromatography analysis of an extract extracted with an ethanol and toluene azeotropic mixture defined by JIS K 6229. The RB/RA of the rosin acid and rosin acid salt is, for example, 0.10, 0.20, 0.30, 0.40, 0.50, 0.60, or 0.70, and may be within a range between any two of the numerical values exemplified here. By adjusting the type and amount of the rosin acid and/or the rosin acid salt, the RB/RA of a dip-molded body of the obtained chloroprene-based polymer latex composition can be adjusted.

The emulsifier can also comprise emulsifiers and dispersants other than rosin acids and rosin acid salts, and as emulsifiers and dispersants other than rosin acids and rosin acid salts, cationic, anionic, and nonionic emulsifiers and dispersants can be used. In one embodiment of the present invention, as the emulsifier used in the emulsion polymerization step, a rosin acid and/or a rosin acid salt and an anionic emulsifier or dispersant can be comprised. As the anionic emulsifier or dispersant, from the viewpoint of stabilizing the chloroprene-based polymer latex when a pH adjuster is

added, it is preferable to use sulfate-based or sulfonate-based anionic emulsifiers or dispersants in combination. Specific examples include alkyl sulfonates having 8 to 20 carbon atoms, alkyl aryl sulfates, a condensate of sodium β-naphthalenesulfonate and formaldehyde, and sodium alkyldiphenylether disulfonate. The addition amount of the anionic emulsifier or dispersant can be 0.05 to 5 parts by mass with respect to 100 parts by mass of the raw material monomer. The addition amount of the anionic emulsifier or dispersant is, for example, 0.05, 0.1, 0.2, 0.3, 0.5, 1.0, 1.5, 2.0, 2.5, 3.0, 3.5, 4.0, 4.5, or 5.0 parts by mass with respect to 100 parts by mass of the raw material monomer, and may be within a range between any two of the numerical values exemplified here.

The pH of an aqueous emulsion at the start of emulsion polymerization is preferably 10.5 to 13.5. The aqueous emulsion refers to a mixed liquid of a chain transfer agent, monomers (chloroprene, 2,3-dichloro-1,3-butadiene, and the like) immediately before the start of emulsion polymerization, but also includes cases where its composition changes due to post-addition or divided addition of each component. When the pH of the aqueous emulsion at the start of emulsion polymerization is 10.5 or more, the polymerization reaction can be controlled more stably. When the pH is 13.5 or less, an excessive increase in viscosity during polymerization is suppressed, and the polymerization reaction can be controlled more stably.

The polymerization temperature of emulsion polymerization is preferably in the range of 5 to 55°C. It is preferable because the emulsion does not freeze at 5°C or higher, and there is no evaporation or boiling of chloroprene monomer at 55°C or lower.

As the polymerization initiator, potassium persulfate, benzoyl peroxide, ammonium persulfate, hydrogen peroxide, and the like, which are used in ordinary radical polymerization, can be used.

The polymerization conversion rate is preferably in the range of 50 to 95%. The polymerization reaction is stopped by adding a polymerization inhibitor. When the polymerization conversion rate is 50% or more, the toluene-insoluble content tends to increase, and the tensile strength at break of the obtained dip-molded body tends to be high. It is also advantageous from the viewpoint of production cost. When the polymerization conversion rate is less than 95%, a decrease in polymerization reactivity due to a decrease in unreacted monomers can be avoided, and a decrease in productivity can be avoided.

Examples of the polymerization inhibitor include diethylhydroxylamine, thiodiphenylamine, 4-tert-butylcatechol, 2,2'-methylenebis-4-methyl-6-tert-butylphenol, and the like. Unreacted monomers after completion of emulsion polymerization can be removed by a conventional method such as vacuum distillation.

In addition, to the chloroprene-based polymer latex obtained by the production method of one embodiment of the present invention, a freeze stabilizer, an emulsion stabilizer, a viscosity adjuster, an antioxidant, a preservative, and the like can be arbitrarily added after polymerization within a range that does not impair the effects of the present invention.

As one example, in the polymerization step, polymerization can be started after charging all of the raw material monomers and chemicals used in the polymerization step into a polymerization vessel before the start of polymerization, or at least a part of the raw material monomers and/or chemicals used in the polymerization step can be charged into the polymerization vessel before the start of polymerization, and the remainder can be added portion-wise after the start of polymerization.

In the polymerization step, when at least a part of the raw material monomers and chemicals is charged into the polymerization vessel before the start of polymerization, and the remaining raw material monomers and/or chemicals are added after the start of polymerization, the remaining raw material monomers and/or chemicals can be added portion-wise (either in a single portion or in multiple portions), or can be added continuously at a constant flow rate. In one embodiment of the present invention, at least a part of the raw material monomers can be added portion-wise after the start of polymerization. As one example, when chloroprene and 2,3-dichloro-1,3-butadiene are used as raw material monomers, a part of chloroprene can be added portion-wise after the start of polymerization.

As one example, the portion-wise addition of the remaining raw material monomers and/or chemicals can be started when the polymerization rate of the raw material monomers charged before the start of polymerization reaches 50 to 95%. The polymerization rate at the start of portion-wise addition can be, for example, 50, 55, 60, 65, 70, 75, 80, 85, 90, or 95%, and may be within a range between any two of the numerical values exemplified here.

When at least a part of the raw material monomers is added after the start of polymerization, 50 parts by mass or less of 100 parts by mass of all raw material monomers used in the polymerization step can be added after the start of polymerization. In this case, the amount of raw material monomers added after the start of polymerization can be, for example, 0, 5, 10, 15, 20, 25, 30, 35, 40, 45, or 50 parts by mass, and may be within a range between any two of the numerical values exemplified here. As one example, the portion-wise addition of a raw material monomer, for example, chloroprene, can be performed continuously over 30 to 300 minutes.

Hereinafter, a method for producing the chloroprene-based polymer latex (A) according to the second embodiment of the present invention will be described. The method for producing the chloroprene-based polymer latex (A) according to the second embodiment of the present invention can include a polymerization step and a mixing step.

The method for producing the chloroprene-based polymer latex (A) according to the second embodiment of the present invention can include a polymerization step of polymerizing a raw material monomer comprising chloroprene to

obtain a chloroprene-based polymer latex, and the polymerization step can include a polymerization step of polymerizing a raw material monomer comprising chloroprene to obtain a latex comprising a chloroprene-based polymer α, and a polymerization step of polymerizing a raw material monomer comprising chloroprene to obtain a latex comprising a chloroprene-based polymer β.

In the polymerization step, it is preferable to adjust the amount of a chain transfer agent and the like according to the target weight average molecular weight. In addition, regarding the types and amounts of chemicals, polymerization conditions, and the like in the polymerization step of the second embodiment, they can be as described in the polymerization step in the first embodiment described above.

The method for producing the chloroprene-based polymer latex (A) according to the second embodiment of the present invention can include a mixing step of mixing two or more types of latexes to obtain the chloroprene-based polymer latex (A). In the mixing step, a latex comprising a chloroprene-based polymer α having a weight average molecular weight of 5,000 to 80,000 and a latex comprising a chloroprene-based polymer β having a weight average molecular weight of 200,000 to 1,500,000 can be mixed to obtain the chloroprene-based polymer latex (A). In the mixing step, two or more types of chloroprene-based polymer latexes can be mixed by a known method. In the mixing step, for example, the chloroprene-based polymer latex (A) may be obtained by stirring and mixing using a paddle blade at 30 to 300 rpm for 20 seconds to 3 minutes, for example, at 100 rpm for 1 minute.

Hereinafter, a method for producing the chloroprene-based polymer latex (A) according to the third embodiment of the present invention will be described. The method for producing the chloroprene-based polymer latex (A) according to the third embodiment of the present invention can include:

a first polymerization step of polymerizing a raw material monomer comprising chloroprene to polymerize a chloroprene-based polymer α, and a second polymerization step of polymerizing a chloroprene-based polymer β in the presence of the chloroprene-based polymer α, or,
a first polymerization step of polymerizing a raw material monomer comprising chloroprene to polymerize a chloroprene-based polymer β, and a second polymerization step of polymerizing a chloroprene-based polymer α in the presence of the chloroprene-based polymer β.

In the first polymerization step, it is preferable to adjust the amount of a chain transfer agent and the like according to the target weight average molecular weight. In addition, in the method for producing a chloroprene-based polymer latex according to the third embodiment of the present invention, a polymerization inhibitor may not be used in the first polymerization step. Otherwise, the types and amounts of chemicals, polymerization conditions, and the like in the first polymerization step are as described in the polymerization step in the first embodiment described above.

The third embodiment of the present invention can include a second polymerization step of polymerizing a chloroprene-based polymer β in the presence of a chloroprene-based polymer α, or a second polymerization step of polymerizing a chloroprene-based polymer α in the presence of a chloroprene-based polymer β.

In the second polymerization step, the chloroprene-based polymer α (or the chloroprene-based polymer β) may be added to a polymerization solution in any form, and for example, a polymerization solution in which the chloroprene-based polymer α (or the chloroprene-based polymer β) has been polymerized may be used as is. As one example, when the chloroprene-based polymer α (or the chloroprene-based polymer β) is polymerized by emulsion polymerization, a latex comprising the chloroprene-based polymer α (or the chloroprene-based polymer β) may be added to a polymerization solution of the chloroprene-based polymer β or the chloroprene-based polymer α. Also, for example, the chloroprene-based polymer α (or the chloroprene-based polymer β) may be precipitated from a polymerization solution in which the chloroprene-based polymer α (or the chloroprene-based polymer β) has been polymerized, and the precipitated chloroprene-based polymer α (or the chloroprene-based polymer β) may be added to a polymerization solution of the chloroprene-based polymer β (or the chloroprene-based polymer α). As one example, when the chloroprene-based polymer α (or the chloroprene-based polymer β) is polymerized by emulsion polymerization, the chloroprene-based polymer α (or the chloroprene-based polymer β) may be precipitated with methanol and added to a polymerization solution of the chloroprene-based polymer β (or the chloroprene-based polymer α), or a rubber fraction (chloroprene-based polymer α (or chloroprene-based polymer β)) obtained by freeze-drying a latex comprising the chloroprene-based polymer α (or the chloroprene-based polymer β) may be added to a polymerization solution of the chloroprene-based polymer β (or the chloroprene-based polymer α).

In the second polymerization step, when polymerizing the chloroprene-based polymer β, when the charged amount of chloroprene monomer (or raw material monomer) is 100 parts by mass, the charged amount of the chloroprene-based polymer α can be 5.0 to 60.0 parts by mass. The charged amount of the chloroprene-based polymer α is, for example, 5.0, 10.0, 15.0, 20.0, 25.0, 30.0, 35.0, 40.0, 50.0, or 60.0 parts by mass, and may be within a range between any two of the numerical values exemplified here. Also, in the second polymerization step, when polymerizing the chloroprene-based polymer α, when the charged amount of chloroprene monomer (or raw material monomer) is 100 parts by mass, the charged amount of the chloroprene-based polymer β can be 40.0 to 95.0 parts by mass. The charged amount of the

chloroprene-based polymer β is, for example, 40.0, 50.0, 60.0, 65.0, 70.0, 75.0, 80.0, 85.0, 90.0, or 95.0 parts by mass, and may be within a range between any two of the numerical values exemplified here.

In the second polymerization step, the type and charged amount of each monomer are preferably adjusted so that the content rate of each monomer unit in the obtained chloroprene-based polymer latex (A) is within the numerical ranges described above. In addition, regarding the types and amounts of chemicals, polymerization conditions, and the like in the second polymerization step, they can be as described in the polymerization step described above.

A chloroprene-based polymer latex comprising a chloroprene-based polymer α having a weight average molecular weight of 5,000 to 80,000 and/or a chloroprene-based polymer β having a weight average molecular weight of 200,000 to 1,500,000, obtained by the above production method, can be used as the chloroprene-based polymer latex (A) according to the present invention.

1.3 Metal Oxide (B)

The chloroprene-based polymer latex composition according to the present invention comprises 0.3 to 15.0 parts by mass of a metal oxide (B) with respect to 100 parts by mass of the solid content of the chloroprene-based polymer latex (A). The content of the metal oxide (B) is, for example, 0.3, 0.5, 1.0, 2.0, 3.0, 4.0, 5.0, 6.0, 7.0, 8.0, 9.0, 10.0, 11.0, 12.0, 13.0, 14.0, or 15.0 parts by mass, and may be within a range between any two of the numerical values exemplified here. When the content of the metal oxide (B) is the above lower limit or more, the tensile strength at break improves due to a crosslinking effect between polymers. Also, when the content of the metal oxide (B) is the above upper limit or less, a dip-molded body excellent in flexibility can be obtained.

The type of the metal oxide (B) is not particularly limited, and can be at least one selected from a group consisting of zinc oxide, lead oxide, trilead tetraoxide, magnesium oxide, aluminum oxide, iron oxide, beryllium oxide, and titanium oxide. The metal oxide preferably comprises zinc oxide. Zinc oxide is generally considered to function as a scavenger for chlorine atoms released from a chloroprene-based polymer. As the metal oxide (B), one type may be used, or two or more types may be used in combination.

1.4 Vulcanization Accelerator (C)

The chloroprene-based polymer latex composition according to the present invention comprises 0.02 to 1.50 parts by mass of a vulcanization accelerator (C) with respect to 100 parts by mass of the solid content of the chloroprene-based polymer latex (A). The content of the vulcanization accelerator (C) is, for example, 0.02, 0.04, 0.06, 0.08, 0.10, 0.20, 0.30, 0.40, 0.50, 0.60, 0.70, 0.80, 0.90, 1.00, 1.10, 1.20, 1.30, 1.40, or 1.50 parts by mass, and may be within a range between any two of the numerical values exemplified here. The chloroprene-based polymer latex composition according to the present invention can provide a dip-molded body having both excellent flexibility and tensile strength at break, while reducing allergy risks and costs, by comprising a specific amount of the vulcanization accelerator (C) that is smaller than in conventional chloroprene-based polymer latex compositions.

The vulcanization accelerator (C) according to the present invention comprises at least one selected from a group consisting of a thiuram-based vulcanization accelerator, a dithiocarbamate-based vulcanization accelerator, a thiourea-based vulcanization accelerator, a guanidine-based vulcanization accelerator, a xanthate-based vulcanization accelerator, and a thiazole-based vulcanization accelerator. As the vulcanization accelerator (C), one type may be used, or two or more types may be used in combination.

Examples of the thiourea-based vulcanization accelerator include a compound having a thiourea structure. Examples of the thiourea-based vulcanization accelerator include a compound represented by the following general formula.

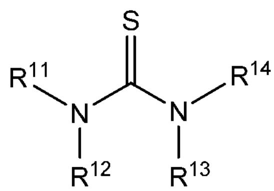

**In** the above general formula, $R^{11}$ to $R^{14}$ can each independently be hydrogen or an organic group, and can be hydrogen or a hydrocarbon group, and the hydrocarbon group can be an alkyl group or an aryl group. Examples of the thiourea-based compound include ethylene thiourea, diethyl thiourea (N,N'-diethyl thiourea), trimethyl thiourea, diphenyl thiourea (N,N'-diphenyl thiourea), 1,3-trimethylene-2-thiourea, and the like.

Examples of the thiuram-based vulcanization accelerator include a compound comprising one or more structures represented by the following general formula.

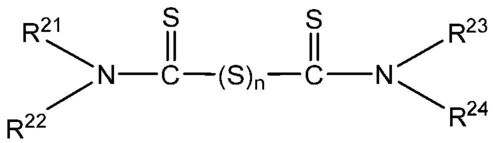

In the above general formula, $R^{21}$, $R^{22}$, $R^{23}$, and $R^{24}$ can each independently be an organic group, and are preferably a hydrocarbon group. The hydrocarbon group can be an alkyl group having 1 to 12 carbon atoms or a cycloalkyl group having 1 to 12 carbon atoms. $R^{21}$ and $R^{22}$, and $R^{23}$ and $R^{24}$ may be linked to each other to form a cyclic structure (for example, a cycloalkyl group). n can be an integer of 1 or more, can be 1 to 4, is preferably 1 or 2, and more preferably 2.

Examples of the thiuram-based vulcanization accelerator include tetramethylthiuram disulfide (TMTD), tetraethylthiuram disulfide, tetrabutylthiuram disulfide, tetrakis(2-ethylhexyl)thiuram disulfide, tetramethylthiuram monosulfide, dipentamethylenethiuram tetrasulfide, and the like.

Examples of the dithiocarbamate-based vulcanization accelerator include a compound composed of an ion represented by the following general formula and a metal ion.

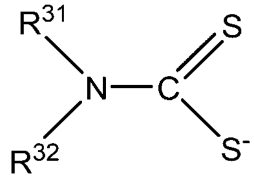

**In** the above general formula, $R^{31}$ to $R^{32}$ can each independently be an organic group, and can be a hydrocarbon group. The hydrocarbon group can be an alkyl group, an aryl group, or an aralkyl group, and $R^{31}$ and $R^{32}$ may be linked to form a cycloalkyl group. Examples of the metal ion include zinc, sodium, copper, iron, nickel, tellurium, and the like. Examples of the dithiocarbamate-based vulcanization accelerator include sodium dibutyldithiocarbamate, zinc dimethyldithiocarbamate, zinc diethyldithiocarbamate, zinc N-ethyl-N-phenyldithiocarbamate, zinc N-pentamethylenedithiocarbamate, copper dimethyldithiocarbamate, ferric dimethyldithiocarbamate, tellurium diethyldithiocarbamate, and the like.

Examples of the guanidine-based compound include a compound having a guanidine skeleton. Examples of the guanidine-based compound include 1,3-diphenylguanidine, 1,3-di-o-tolylguanidine, 1-o-tolylbiguanide, di-o-tolylguanidine salt of dicathechol borate, and the like.

Examples of the xanthate-based vulcanization accelerator include a compound composed of an ion represented by the following general formula and a metal ion.

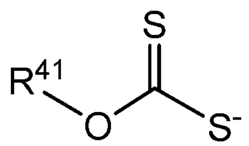

In the above general formula, $R^{41}$ can be an organic group, and can be a hydrocarbon group. The hydrocarbon group can be an alkyl group. Examples of the metal ion include zinc, sodium, copper, nickel, tellurium, and the like. Examples of the xanthate-based vulcanization accelerator include zinc butylxanthate, zinc isopropylxanthate, and the like.

Examples of the thiazole-based compound include a compound having a thiazole skeleton, and it more preferably has a benzothiazole skeleton. Examples of the thiazole-based compound include 2-mercaptobenzothiazole, di-2-benzothiazolyl disulfide, zinc salt of 2-mercaptobenzothiazole, cyclohexylamine salt of 2-mercaptobenzothiazole, 2-(4'-morpholinodithio)benzothiazole, N-cyclohexylbenzothiazole-2-sulfenamide, N-cyclohexyl-2-benzothiazolylsulfenamide, N-oxydiethylene-2-benzothiazolylsulfenamide, and the like.

1.5 Sulfur (D)

The chloroprene-based polymer latex composition according to the present invention comprises 0.01 to 0.75 parts by mass of sulfur (D) with respect to 100 parts by mass of the solid content of the chloroprene-based polymer latex (A). The content of sulfur (D) is, for example, 0.01, 0.02, 0.03, 0.05, 0.10, 0.15, 0.20, 0.25, 0.30, 0.35, 0.40, 0.45, 0.50, 0.55, 0.60, 0.65, 0.70, or 0.75 parts by mass, and may be within a range between any two of the numerical values exemplified here. The chloroprene-based polymer latex composition according to the present invention can provide a dip-molded body having both excellent flexibility and tensile strength at break, while reducing allergy risks and costs, by comprising a specific amount of sulfur (D) that is smaller than in conventional chloroprene-based polymer latex compositions.

1.6 Heteroaromatic Ring Compound (E)

The chloroprene-based polymer latex composition according to the present invention comprises 0.1 to 10.0 parts by mass of a heteroaromatic ring compound (E) with respect to 100 parts by mass of the solid content of the chloroprene-based polymer latex (A).

The heteroaromatic ring compound (E) can be represented by general formula (1) and has a benzimidazole structure. A compound having this structure may mainly be adopted as a secondary antioxidant in a formulation of a rubber composition.

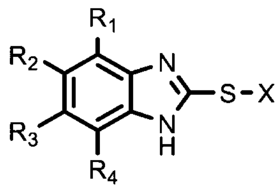

(1)

In general formula (1), X of a mercapto group represents a hydrogen atom or a metal atom. X can be a hydrogen atom, resulting in a thiol group. X can also be a metal atom, and examples of the metal atom include zinc, sodium, copper, nickel, and tellurium, among which zinc is preferable.

$R_1$ to $R_4$ in general formula (1) each represent a hydrogen atom, an optionally substituted alkyl group, an optionally substituted ether group, a nitro group, an amino group, or a carboxyl group. $R_1$ to $R_4$ may be the same or different from each other. Also, as the heteroaromatic ring compound (E), one type may be used, or two or more types may be used in combination.

Examples of the heteroaromatic ring compound (E) include 2-mercaptobenzimidazole, 5-methyl-2-mercaptobenzimidazole, 4-methyl-2-mercaptobenzimidazole, 5-methoxy-2-mercaptobenzimidazole, 4-methoxy-2-mercaptobenzimidazole, 5-nitro-2-mercaptobenzimidazole, 5-amino-2-mercaptobenzimidazole, 5-carboxy-2-mercaptobenzimidazole, or a zinc salt of 2-mercaptobenzimidazole, and the like. Among these, 2-mercaptobenzimidazole, 5-methyl-2-mercaptobenzimidazole, 4-methyl-2-mercaptobenzimidazole, 5-methoxy-2-mercaptobenzimidazole, 4-methoxy-2-mercaptobenzimidazole, and a zinc salt of 2-mercaptobenzimidazole are preferable.

The addition amount of the heteroaromatic ring compound (E) is preferably 0.1 to 10.0 parts by mass with respect to 100 parts by mass of the solid content of the chloroprene-based polymer latex (A) comprised in the chloroprene-based polymer latex composition. The content of the heteroaromatic ring compound (E) is, for example, 0.1, 0.2, 0.3, 0.4, 0.5, 1.0, 2.0, 3.0, 4.0, 5.0, 6.0, 7.0, 8.0, 9.0, or 10.0 parts by mass, and may be within a range between any two of the numerical values exemplified here. When the content of the heteroaromatic ring compound (E) is the above lower limit or more, a dip-molded body exhibits very high tensile strength at break. When the content of the heteroaromatic ring compound (E) is the above upper limit or less, the stability of the chloroprene-based polymer latex composition is ensured. By setting the content of the heteroaromatic ring compound (E) within the above numerical range, the flexibility and tensile strength at break of a dip-molded body can be highly improved. As the heteroaromatic ring compound (E), one type may be used, or two or more types may be used in combination.

1.7 Antioxidant

The chloroprene-based polymer latex composition according to the present invention can comprise 0.5 to 10.0 parts by mass of an antioxidant with respect to 100 parts by mass of the solid content of the chloroprene-based polymer latex (A). The type of antioxidant is not particularly limited, and a phenol-based antioxidant, an amine-based antioxidant, a heat-resistant antioxidant (anti-aging agent), an ozone-resistant antioxidant, and the like can be used (here, the heteroaromatic ring compound (E) can be excluded). When an obtained dip-molded body is used for medical glove applications, a phenol-based antioxidant can be adopted from the viewpoint of color tone, texture, and hygiene of the dip-molded body, and in particular, a hindered phenol-based antioxidant is preferable because the above effects are strong. Examples of the hindered phenol-based antioxidant include 2,2'-methylenebis(4-ethyl-6-t-butylphenol), 2,2'-methylenebis(4-methyl-6-t-butylphenol), 4,4'-butylidene(3-methyl-6-t-butylphenol), 4,4'-thiobis(3-methyl-6-t-butylphenol), a butylated reaction product of p-cresol and dicyclopentadiene, 2,5'-di-t-butylhydroquinone, and 2,5'-di-t-amylhydroquinone. Among these, a butylated reaction product of p-cresol and dicyclopentadiene is desirable from the viewpoint that it is generally preferable in an aqueous material. As the antioxidant, one type may be used, or two or more types may be used in combination.

The content of the antioxidant is preferably 0.5 to 10.0 parts by mass with respect to 100 parts by mass of the solid content of the chloroprene-based polymer latex (A) comprised in the chloroprene-based polymer latex composition. The content of the antioxidant is, for example, 0.5, 1.0, 2.0, 3.0, 4.0, 5.0, 6.0, 7.0, 8.0, 9.0, or 10.0 parts by mass, and may be

within a range between any two of the numerical values exemplified here. When the content of the antioxidant is the above lower limit or more, color tone change of a dip-molded body can be suppressed. When the addition amount of the antioxidant is the above upper limit or less, the stability of the chloroprene-based polymer latex composition is ensured. From the viewpoint of the balance of physical properties such as flexibility and tensile strength at break of an obtained dip-molded body, the addition amount of the antioxidant is more preferably 0.5 to 5.0 parts by mass.

2. Physical Properties of Chloroprene-based Polymer Latex Composition

<Toluene Swelling Degree>

In a chloroprene-based polymer latex composition according to one embodiment of the present invention, a toluene swelling degree of a dip-molded body obtained by molding the chloroprene-based polymer latex composition by a dip coagulation method and heat-drying the molded product at 140°C for 60 minutes is preferably 12.0 or less, and more preferably 10.0 or less. Also, the toluene swelling degree is represented by Equation (1).

$$\text{Equation (1): Toluene swelling degree} = 1 + (W2/W1 - 1)(P1/P2)(1/r)$$

In Equation (1), W1 represents a mass of the dip-molded body before immersing the dip-molded body in toluene, W2 represents a mass of the dip-molded body after immersing the dip-molded body in toluene, P1 represents a specific gravity of a chloroprene-based polymer component in the chloroprene-based polymer latex (A), P2 represents a specific gravity of toluene, and r represents a mass fraction of the chloroprene-based polymer component in the dip-molded body. Here, the chloroprene-based polymer component in the chloroprene-based polymer latex (A) can be obtained by mixing the chloroprene-based polymer latex (A) with methanol to precipitate it, followed by filtration and drying.

The specific gravity of the chloroprene-based polymer component in the chloroprene-based polymer latex (A) and the specific gravity of toluene can be those measured at 20°C, and P2 can be 0.867. P1 can be the specific gravity of the chloroprene-based polymer component in the chloroprene-based polymer latex (A) measured at 20°C, and P1 can be 1.23.

The toluene swelling degree of a dip-molded body of the chloroprene-based polymer latex composition is, for example, 1.0, 2.0, 3.0, 4.0, 5.0, 6.0, 7.0, 8.0, 9.0, 10.0, 11.0, or 12.0, and may be within a range between any two of the numerical values exemplified here.

The toluene swelling degree of a dip-molded body of the chloroprene-based polymer latex composition is considered to be related to the degree of crosslinking of the chloroprene-based polymer latex, and it is presumed that the higher the degree of crosslinking, the lower the swelling degree tends to be. The chloroprene-based polymer latex according to the present invention becomes a dip-molded body having more excellent tensile strength at break and flexibility by setting the toluene swelling degree to 12.0 or less.

The toluene swelling degree can be measured by the method described in Examples. The toluene swelling degree can be adjusted by appropriately selecting a method for producing the chloroprene-based polymer latex composition, and as one example, a chloroprene-based polymer latex composition having a toluene swelling degree of 12.0 or less can be obtained by a production method including a polymerization step of polymerizing a chloroprene-based polymer $\beta$ (or a chloroprene-based polymer $\alpha$) in the presence of a chloroprene-based polymer $\alpha$ (or a chloroprene-based polymer $\beta$), as described above.

<Ratio RB/RA of Total Peak Area RB of Abietic Acid, Neoabietic Acid, Palustric Acid, Levopimaric Acid, and Salts Thereof to Total Peak Area RA of Dehydroabietic Acid, Pimaric Acid, Isopimaric Acid, Dihydroabietic Acid, and Salts Thereof>

In a chloroprene-based polymer latex composition according to one embodiment of the present invention, a ratio RB/RA is preferably 0.10 or more, more preferably 0.10 or more and 0.70 or less, and still more preferably 0.15 or more and 0.50 or less, where RA is a total peak area of dehydroabietic acid, pimaric acid, isopimaric acid, dihydroabietic acid, and salts thereof, and RB is a total peak area of abietic acid, neoabietic acid, palustric acid, levopimaric acid, and salts thereof, obtained from a gas chromatography analysis of an extract extracted with an ethanol and toluene azeotropic mixture defined by JIS K 6229 from a dip-molded body obtained by molding the chloroprene-based polymer latex composition by a dip coagulation method and heat-drying the molded product at 140°C for 60 minutes. The RB/RA is, for example, 0.10, 0.20, 0.30, 0.40, 0.50, 0.60, or 0.70, and may be within a range between any two of the numerical values exemplified here. By setting the RB/RA within the above numerical range, the tensile strength at break of a dip-molded body can be further improved.

The RB/RA can be determined by molding a chloroprene-based polymer latex composition by a dip coagulation

method, heat-drying the molded product at 140°C for 60 minutes to obtain a dip-molded body, putting the dip-molded body into a flask equipped with a condenser, extracting with an ethanol and toluene azeotropic mixture defined by JIS K 6229, performing hydrochloric acid treatment on the extract, and then performing gas chromatography analysis, and can be calculated by the method described in Examples. The RB/RA can be controlled by adjusting the types and blending ratio of rosin acids and rosin acid salts added as an emulsifier during the production of the chloroprene-based polymer latex.

In a chloroprene-based polymer latex composition according to one embodiment of the present invention, a 100% elongation modulus, measured based on JIS K 6251, of a dip-molded body obtained by molding the chloroprene-based polymer latex composition by a dip coagulation method and heat-drying the molded product at 140°C for 60 minutes is preferably 0.65 MPa or less, and more preferably 0.60 MPa. The 100% elongation modulus is, for example, 0.30, 0.35, 0.4, 0.45, 0.50, 0.55, 0.60, or 0.65 MPa, and may be within a range between any two of the numerical values exemplified here.

In a chloroprene-based polymer latex composition according to one embodiment of the present invention, a tensile strength at break, measured based on JIS K 6251, of a dip-molded body obtained by molding the chloroprene-based polymer latex composition by a dip coagulation method and heat-drying the molded product at 140°C for 60 minutes is preferably 19.0 MPa or more, and more preferably 20.0 MPa. The tensile strength at break is, for example, 19.0, 20.0, 21.0, 22.0, 23.0, 24.0, or 25.0 MPa, and may be within a range between any two of the numerical values exemplified here.

The tensile strength at break and 100% elongation modulus of a dip-molded body of the chloroprene-based polymer latex composition can be controlled by adjusting the type and amount of formulation of the chloroprene-based polymer latex composition, as well as the polymerization formulation and conditions, weight average molecular weight, toluene-insoluble content, and the like of the chloroprene-based polymer latex used.

3. Method for Producing Chloroprene-based Polymer Latex Composition

A method for producing a chloroprene-based polymer latex composition according to one embodiment of the present invention can include a raw material mixing step of mixing raw materials including a chloroprene-based polymer latex (A), a metal oxide (B), a vulcanization accelerator (C), sulfur (D), and a heteroaromatic ring compound (E), as well as other necessary chemicals.

In the mixing step, an aqueous dispersion comprising the metal oxide (B), the vulcanization accelerator (C), the sulfur (D), and the heteroaromatic ring compound (E), as well as other necessary chemicals, can be prepared in advance, and then the chloroprene-based polymer latex (A) and the aqueous dispersion can be mixed.

The mixing step can be performed with a known mixing device such as a ball mill.

4. Dip-molded Body (Dip-molded Coating and Film)

A dip-molded body according to one embodiment of the present invention is a dip-molded body of the above-described chloroprene-based polymer latex composition. The dip-molded body according to one embodiment of the present invention is obtained by dip-molding the chloroprene-based polymer latex composition according to the present invention, either alone or blended with another chloroprene-based polymer latex composition. It has a low modulus value at 100% elongation, is flexible, and is excellent in mechanical properties such as strength and elongation, and for example, can have the above-described 100% elongation modulus and/or tensile strength at break. The dip-molded body can be suitably used as gloves for industrial or general household use, medical gloves, balloons, catheters, and boots.

A thickness (for example, a minimum thickness) of the dip-molded body may be 0.01 to 0.50 mm. The thickness of the dip-molded body is, for example, 0.01, 0.05, 0.10, 0.20, 0.30, 0.40, or 0.50 mm, and may be within a range between any two of the numerical values exemplified here. The thickness of the dip-molded body can be adjusted by a time for immersing a mold in the polymer latex composition, a solid content concentration of the chloroprene-based polymer latex composition, and the like. When it is desired to make the thickness of the dip-molded body thin, the immersion time may be shortened, or the solid content concentration of the chloroprene-based polymer latex composition may be lowered.

5. Method for Producing Dip-molded Body

A method for producing a dip-molded body according to one embodiment of the present invention can include a dip-molding step of dip-molding the chloroprene-based polymer latex composition according to the present invention to obtain a dip-molded body.

Examples of the dip-molding method according to one embodiment of the present invention include a dip coagulation method, a simple dipping method, a heat-sensitive dipping method, an electrodeposition method, and the like. From the viewpoint of ease of production and ease of obtaining a dip-molded body with a uniform thickness, the dip coagulation method can be used. Specifically, a ceramic mold coated with a calcium-based coagulant solution is immersed in a composition for forming a dip-molded body, and the composition for forming a dip-molded body is

coagulated. Then, after removing water-soluble impurities by leaching, it is dried, and further heated and vulcanized to form a dip-molded coating (rubber coating), after which the dip-molded coating is released from the mold. Thereby, a film-like dip-molded body can be obtained.

A method for producing a dip-molded body according to one embodiment of the present invention can include a drying step of heat-drying the dip-molded body.

A heat-drying temperature may be appropriately set according to the composition of the chloroprene-based polymer latex composition, and may be 120 to 180°C. The heating temperature is preferably 120 to 150°C. The heating temperature is, for example, 120, 130, 140, 150, 160, 170, 180, 190, 200, 210, or 220°C, and may be within a range between any two of the numerical values exemplified here. A heating time may be appropriately set according to the composition of the chloroprene-based polymer latex composition, a shape of an unvulcanized molded body, and the like, and may be 10 to 300 minutes. The heating time is, for example, 10, 20, 30, 40, 50, 60, 70, 80, 90, 100, 110, 120, 130, 140, 150, 160, 170, 180, 190, 200, 210, 220, 230, 240, 250, 260, 270, 280, 290, or 300 minutes, and may be within a range between any two of the numerical values exemplified here. As one example, a dip-molded body according to one embodiment of the present invention can be subjected to a heat-drying treatment at 140°C for 60 minutes.

## EXAMPLES

Hereinafter, the present invention will be described in more detail based on Examples, but the present invention is not to be construed as being limited thereto.

First, a method for preparing a chloroprene-based polymer latex used in Examples will be described.

### <Preparation of Chloroprene-based Polymer Latex A-1>

(Polymerization Step of Chloroprene-based Polymer α-1)

Into a polymerization vessel with an internal volume of 30 L, 64 parts by mass of chloroprene (monomer), 9 parts by mass of 2,3-dichloro-1,3-butadiene, 90 parts by mass of pure water, 4.5 parts by mass of a conjugated resin acid-based rosin acid (trade name "Hartall R-WW", manufactured by Harima Chemicals Group, Inc.), 3.4 parts by mass of n-dodecyl mercaptan, 1.6 parts by mass of potassium hydroxide, 0.5 parts by mass of a sodium salt of β-naphthalenesulfonic acid formalin condensate (product name "Demol N", manufactured by Kao Corporation), and 0.5 parts by mass of sodium bisulfite were added. Polymerization was performed by continuously adding a 0.35% by mass aqueous solution of potassium persulfate as a polymerization initiator under a nitrogen stream at a polymerization temperature of 35°C. When the polymerization rate of the charged monomers reached 80%, 27 parts by mass of chloroprene (monomer) was continuously added over 100 minutes. When the polymerization rate of the initially charged monomers and the continuously added monomers reached 91%, the addition of the aqueous potassium persulfate solution was stopped to terminate the polymerization, thereby obtaining a polymerization liquid. Unreacted monomers were removed from the polymerization liquid by vacuum distillation, and a latex comprising chloroprene-based polymer α-1 with a solid content concentration of 60% by mass was obtained by concentration.

(Precipitation of Chloroprene-based Polymer by Methanol)

The obtained latex comprising chloroprene-based polymer α-1 was mixed with a large amount of methanol to precipitate chloroprene-based polymer α-1, which was then filtered and dried to obtain a chloroprene-based polymer. This was used for the preparation of chloroprene-based polymer β-1, β-23, and chloroprene-based polymer latex A-14 described later.

(Polymerization Step of Chloroprene-based Polymer β-1)

Into a polymerization vessel with an internal volume of 30 L, 28.3 parts by mass of the chloroprene-based polymer precipitated from the latex comprising chloroprene-based polymer α-1, 91 parts by mass of chloroprene (monomer), 9 parts by mass of 2,3-dichloro-1,3-butadiene, 110 parts by mass of pure water, 5.8 parts by mass of a conjugated resin acid-based rosin acid (product name "Hartall R-WW", manufactured by Harima Chemicals Group, Inc.), 2 parts by mass of potassium hydroxide, 0.5 parts by mass of a sodium salt of β-naphthalenesulfonic acid formalin condensate (product name "Demol N", manufactured by Kao Corporation), 0.5 parts by mass of sodium bisulfite, and 0.03 parts by mass of thiourea dioxide were added. Polymerization was performed by continuously adding a 0.35% by mass aqueous solution of potassium persulfate as a polymerization initiator under a nitrogen stream at a polymerization temperature of 14°C. When the polymerization rate reached 85%, 0.1 parts by mass of diethylhydroxylamine as a polymerization inhibitor was added to terminate the polymerization, thereby obtaining a polymerization liquid. Unreacted monomers were removed from the

polymerization liquid by vacuum distillation, and chloroprene-based polymer latex A-1 comprising chloroprene-based polymer β-1 with a solid content concentration of 60% by mass was obtained by concentration.

Chloroprene-based polymer latex A-1 was sampled at 50 ml, and the remaining chloroprene-based polymer latex A-1 was used to prepare a dip-molded body for evaluation.

The sampled chloroprene-based polymer latex was mixed with a large amount of methanol to precipitate a chloroprene-based polymer, which was then filtered and dried to obtain a sample of the chloroprene-based polymer. The weight average molecular weight of the chloroprene-based polymer latex was measured from the obtained sample. When the weight average molecular weight of chloroprene-based polymer latex A-1 was measured, a peak derived from chloroprene-based polymer α-1 having a weight average molecular weight of 5,000 to 80,000, and a peak derived from chloroprene-based polymer β-1 having a weight average molecular weight of 200,000 to 1,500,000 were confirmed. In addition, the toluene-insoluble content of a chloroprene-based polymer rubber obtained by freeze-drying the chloroprene-based polymer latex was measured. Each measurement method will be described later.

<Preparation of Chloroprene-based Polymer Latex A-13>

(Synthesis of Chloroprene-based Polymer β-13)

Into a polymerization vessel with an internal volume of 30 L, 91 parts by mass of chloroprene (monomer), 9 parts by mass of 2,3-dichloro-1,3-butadiene, **110** parts by mass of pure water, 4.7 parts by mass of a conjugated resin acid-based rosin acid (product name "Hartall R-WW", manufactured by Harima Chemicals Group, Inc.), 0.02 parts by mass of n-dodecyl mercaptan, 1.6 parts by mass of potassium hydroxide, 0.4 parts by mass of a sodium salt of β-naphthalenesulfonic acid formalin condensate (product name "Demol N", manufactured by Kao Corporation), and 0.4 parts by mass of sodium bisulfite were added. Polymerization was performed by continuously adding a 0.35% by mass aqueous solution of potassium persulfate as a polymerization initiator under a nitrogen stream at a polymerization temperature of 40°C. When the polymerization rate of the charged monomers reached 85%, 0.01 parts by mass of diethylhydroxylamine as a polymerization inhibitor was added to terminate the polymerization. Unreacted monomers were removed from the polymerization liquid by vacuum distillation, and chloroprene-based polymer latex A-13 comprising chloroprene-based polymer β-13 with a solid content concentration of 60% by mass was obtained by concentration. Analysis of weight average molecular weight and toluene-insoluble content was performed in the same manner as for chloroprene-based polymer latex A-1.

Preparation of Chloroprene-based Polymer Latex A-14>

(Synthesis of Chloroprene-based Polymer β-14)

Into a polymerization vessel with an internal volume of 30 L, 91 parts by mass of chloroprene (monomer), 9 parts by mass of 2,3-dichloro-1,3-butadiene, **110** parts by mass of pure water, 4.7 parts by mass of a conjugated resin acid-based rosin acid (product name "Hartall R-WW", manufactured by Harima Chemicals Group, Inc.), 0.02 parts by mass of n-dodecyl mercaptan, 1.6 parts by mass of potassium hydroxide, 0.4 parts by mass of a sodium salt of β-naphthalenesulfonic acid formalin condensate (product name "Demol N", manufactured by Kao Corporation), 0.4 parts by mass of sodium bisulfite, and 0.03 parts by mass of thiourea dioxide were added. Polymerization was performed by continuously adding a 0.35% by mass aqueous solution of potassium persulfate as a polymerization initiator under a nitrogen stream at a polymerization temperature of 14°C. When the polymerization rate of the charged monomers reached 85%, 0.01 parts by mass of diethylhydroxylamine as a polymerization inhibitor was added to terminate the polymerization. Unreacted monomers were removed from the polymerization liquid by vacuum distillation, and a latex comprising chloroprene-based polymer β-14 with a solid content concentration of 60% by mass was obtained by concentration.

(Preparation of Chloroprene-based Polymer Latex A-14)

The above latex comprising chloroprene-based polymer α-1 and the above latex comprising chloroprene-based polymer β-14 were mixed so that a solid content ratio was 25:75, to obtain chloroprene-based polymer latex A-14. Analysis of weight average molecular weight and toluene-insoluble content was performed in the same manner as for chloroprene-based polymer latex A-1.

<Preparation of Chloroprene-based Polymer Latex A-15>

(Polymerization of Chloroprene-based Polymer α-15)

Into a polymerization vessel with an internal volume of 30 L, 64 parts by mass of chloroprene (monomer), 9 parts by mass of 2,3-dichloro-1,3-butadiene, 77 parts by mass of pure water, 17.6 parts by mass of a gum rosin-based disproportionated rosin aqueous solution (product name "Rondis K-25", solid content 25% by mass, manufactured by Arakawa Chemical Industries, Ltd.), 3.4 parts by mass of n-dodecyl mercaptan, 0.8 parts by mass of potassium hydroxide, 0.5 parts by mass of a sodium salt of β-naphthalenesulfonic acid formalin condensate (product name "Demol N", manufactured by Kao Corporation), and 0.5 parts by mass of sodium bisulfite were added. Polymerization was performed by continuously adding a 0.35% by mass aqueous solution of potassium persulfate as a polymerization initiator under a nitrogen stream at a polymerization temperature of 35°C. When the polymerization rate of the charged monomers reached 80%, 27 parts by mass of chloroprene (monomer) was continuously added over 100 minutes. When the polymerization rate of the initially charged monomers and the continuously added monomers reached 91%, the addition of the aqueous potassium persulfate solution was stopped to terminate the polymerization, thereby obtaining a polymerization liquid. Unreacted monomers were removed from the polymerization liquid by vacuum distillation, and a latex comprising chloroprene-based polymer α-15 with a solid content concentration of 60% by mass was obtained by concentration. The obtained latex comprising chloroprene-based polymer α-15 was mixed with a large amount of methanol to precipitate chloroprene-based polymer α-15, which was then filtered and dried to obtain a chloroprene-based polymer. This was used for the preparation of chloroprene-based polymer β-15 described later.

(Preparation of Chloroprene-based Polymer β-15)

Into a polymerization vessel with an internal volume of 30 L, 28.3 parts by mass of the chloroprene-based polymer precipitated from the latex comprising chloroprene-based polymer α-15, 91 parts by mass of chloroprene (monomer), 9 parts by mass of 2,3-dichloro-1,3-butadiene, 100 parts by mass of pure water, 18.5 parts by mass of a gum rosin-based disproportionated rosin aqueous solution (product name "Rondis K-25", solid content 25% by mass, manufactured by Arakawa Chemical Industries, Ltd.), 0.8 parts by mass of potassium hydroxide, 0.5 parts by mass of a sodium salt of β-naphthalenesulfonic acid formalin condensate (product name "Demol N", manufactured by Kao Corporation), 0.5 parts by mass of sodium bisulfite, and 0.03 parts by mass of thiourea dioxide were added. Polymerization was performed by continuously adding a 0.35% by mass aqueous solution of potassium persulfate as a polymerization initiator under a nitrogen stream at a polymerization temperature of 14°C. When the polymerization rate reached 85%, 0.1 parts by mass of diethylhydroxylamine as a polymerization inhibitor was added to terminate the polymerization, thereby obtaining a polymerization liquid. Unreacted monomers were removed from the polymerization liquid by vacuum distillation, and chloroprene-based polymer latex A-15 comprising chloroprene-based polymer β-15 with a solid content concentration of 60% by mass was obtained by concentration. Analysis of weight average molecular weight and toluene-insoluble content was performed in the same manner as for chloroprene-based polymer latex A-1.

<Preparation of Chloroprene-based Polymer Latex A-17>

(Polymerization of Chloroprene-based Polymer α-17)

Into a polymerization vessel with an internal volume of 30 L, 60 parts by mass of chloroprene (monomer), 8 parts by mass of 2,3-dichloro-1,3-butadiene, 90 parts by mass of pure water, 4.5 parts by mass of a conjugated resin acid-based rosin acid (product name "Hartall R-WW", manufactured by Harima Chemicals Group, Inc.), 3 parts by mass of n-dodecyl mercaptan, 1.5 parts by mass of potassium hydroxide, and 0.5 parts by mass of a sodium salt of β-naphthalenesulfonic acid formalin condensate (product name "Demol N", manufactured by Kao Corporation) were added. Polymerization was performed by continuously adding a 0.35% by mass aqueous solution of potassium persulfate as a polymerization initiator under a nitrogen stream at a polymerization temperature of 40°C. When the polymerization rate of the charged monomers reached 65%, 32 parts by mass of chloroprene (monomer) was continuously added over 100 minutes. When the polymerization rate of the initially charged monomers and the continuously added monomers reached 88%, 0.1 parts by mass of diethylhydroxylamine as a polymerization inhibitor was added to terminate the polymerization, thereby obtaining a polymerization liquid. Unreacted monomers were removed from the polymerization liquid by vacuum distillation, and a latex comprising chloroprene-based polymer α-17 with a solid content concentration of 60% by mass was obtained by concentration.

(Polymerization of Chloroprene-based Polymer β-17)

Into a polymerization vessel with an internal volume of 30 L, 91 parts by mass of chloroprene (monomer), 9 parts by mass of 2,3-dichloro-1,3-butadiene, 90 parts by mass of pure water, 4.5 parts by mass of a conjugated resin acid-based

rosin acid (product name "Hartall R-WW", manufactured by Harima Chemicals Group, Inc.), 0.02 parts by mass of n-dodecyl mercaptan, 1.5 parts by mass of potassium hydroxide, and 0.5 parts by mass of a sodium salt of β-naphthalenesulfonic acid formalin condensate (product name "Demol N", manufactured by Kao Corporation) were added. Polymerization was performed by continuously adding a 0.35% by mass aqueous solution of potassium persulfate as a polymerization initiator under a nitrogen stream at a polymerization temperature of 25°C. When the polymerization rate of the charged monomers reached 82%, 0.01 parts by mass of diethylhydroxylamine as a polymerization inhibitor was added to terminate the polymerization. Unreacted monomers were removed from the polymerization liquid by vacuum distillation, and a latex comprising chloroprene-based polymer β-17 with a solid content concentration of 60% by mass was obtained by concentration.

(Preparation of Chloroprene-based Polymer Latex A-17)

The latex comprising chloroprene-based polymer α-17 and the latex comprising chloroprene-based polymer β-17 were mixed so that a solid content ratio was 25:75, to obtain chloroprene-based polymer latex A-17. Analysis of weight average molecular weight and toluene-insoluble content was performed in the same manner as for chloroprene-based polymer latex A-1.

<Preparation of Chloroprene-based Polymer Latex A-23>

(Synthesis of Chloroprene-based Polymer β-23)

Into a polymerization vessel with an internal volume of 30 L, 85 parts by mass of the chloroprene-based polymer precipitated from the latex comprising chloroprene-based polymer α-1, 9 parts by mass of chloroprene (monomer), 9 parts by mass of 2,3-dichloro-1,3-butadiene, 110 parts by mass of pure water, 8.5 parts by mass of a conjugated resin acid-based rosin acid (product name "Hartall R-WW", manufactured by Harima Chemicals Group, Inc.), 3 parts by mass of potassium hydroxide, 0.7 parts by mass of a sodium salt of β-naphthalenesulfonic acid formalin condensate (product name "Demol N", manufactured by Kao Corporation), 0.7 parts by mass of sodium bisulfite, and 0.03 parts by mass of thiourea dioxide were added. Polymerization was performed by continuously adding a 0.35% by mass aqueous solution of potassium persulfate as a polymerization initiator under a nitrogen stream at a polymerization temperature of 14°C. When the polymerization rate reached 85%, 0.1 parts by mass of diethylhydroxylamine as a polymerization inhibitor was added to terminate the polymerization, thereby obtaining a polymerization liquid. Unreacted monomers were removed from the polymerization liquid by vacuum distillation, and chloroprene-based polymer latex A-23 comprising chloroprene-based polymer β-23 with a solid content concentration of 60% by mass was obtained by concentration.

<Analysis Method of Chloroprene-based Polymer Latex>

Analysis of the chloroprene-based polymer latex was performed by the following method.

(Measurement of Weight Average Molecular Weight of Chloroprene-based Polymer Latex)

The weight average molecular weight was measured under the following measurement conditions by gel permeation chromatography (GPC) using a sample obtained by precipitating each chloroprene-based polymer latex with methanol, filtering, drying the chloroprene-based polymer, as described above, and dissolving it in 20 ml of tetrahydrofuran.

Apparatus name: HLC-8320 (manufactured by Tosoh Corporation)
Column: Three TSKgel GMHHR-H columns in series
Temperature: 40°C
Detection: Differential refractometer
Solvent: Tetrahydrofuran
Calibration curve: Prepared using standard polystyrene (PS).

(Toluene-insoluble Content)

A test piece was obtained by cutting a chloroprene-based polymer rubber, obtained by freeze-drying the obtained chloroprene-based polymer latex, into 2 mm squares. After putting this test piece into a conical beaker, the test piece was dissolved in 80 g of toluene over 16 hours. Subsequently, after performing centrifugation, a gel fraction (insoluble fraction) was separated using a 200-mesh wire net. Thereafter, the gel fraction was dried, and then the mass of the dried material

was measured. The toluene-insoluble content in the chloroprene-based polymer rubber was determined by the following formula, where A g is the chloroprene-based polymer rubber after freeze-drying, and B g is the gel fraction (insoluble fraction) separated from a mixture dissolved in toluene.

$$\text{Toluene-insoluble content (gel fraction)} = B/A \times 100\ (\%)$$

(Example 1)

<Preparation of Chloroprene-based Polymer Latex Composition>

2 parts by mass of Type 2 zinc oxide as a metal oxide (B), 0.5 parts by mass of Nocceler BZ as a vulcanization accelerator (C), 0.25 parts by mass of sulfur (D), 2 parts by mass of Nocrac PBK as an antioxidant, 1 part by mass of a heteroaromatic ring compound (E) represented by chemical formula 2, and 0.1 parts by mass of a sodium salt of β-naphthalenesulfonic acid formalin condensate (product name "Demol N", manufactured by Kao Corporation) were added to water, and mixed for 16 hours at 20°C using a ceramic ball mill to prepare an aqueous dispersion.

The above aqueous dispersion was mixed with chloroprene-based polymer latex A-1, and further, water was added so that a solid content concentration became 30% by mass, to prepare a chloroprene-based polymer latex composition. The obtained chloroprene-based polymer latex composition comprises each chemical in the blending amounts as described in Table 1, with respect to the solid content of the chloroprene-based polymer latex.

<Preparation of Dip-molded Body Film>

A ceramic cylinder with an outer diameter of 50 mm (manufactured by Shinko Corporation) was immersed for 1 second in a coagulant solution prepared by mixing 62 parts by mass of water, 35 parts by mass of calcium nitrate tetrahydrate, and 3 parts by mass of calcium carbonate, and then taken out. After drying for 3 minutes, it was immersed for 2 minutes in the chloroprene-based polymer latex composition prepared by the procedure described above. Thereafter, it was washed with running water at 45°C for 1 minute, and dried at 140°C for 60 minutes to prepare a dip-molded body film (dip-molded coating) for evaluation.

(Examples 2 to 15, Comparative Examples 1 to 8)

A chloroprene-based polymer latex composition and a dip-molded body film (dip-molded coating) for evaluation were prepared in the same manner as in Example 1, except that the type of chloroprene-based polymer latex used was as described in Tables 1 to 3, and the formulation of the aqueous dispersion was adjusted so that the blending amount of each chemical was as shown in Tables 1 to 3.

Metal Oxide (B)

Type 2 zinc oxide: Zinc oxide, manufactured by Sakai Chemical Industry Co., Ltd., (product name "Type 2 zinc oxide")

Vulcanization Accelerator (C)

Nocceler BZ: Zinc dibutyldithiocarbamate, manufactured by Ouchi Shinko Chemical Industrial Co., Ltd., (product name "Nocceler BZ")
Nocceler C: N,N'-diphenylthiourea, manufactured by Ouchi Shinko Chemical Industrial Co., Ltd., (product name "Nocceler C")
Nocceler TET: Tetraethylthiuram disulfide, manufactured by Ouchi Shinko Chemical Industrial Co., Ltd., (product name "Nocceler TET")
Nocceler D: 1,3-diphenylguanidine, manufactured by Ouchi Shinko Chemical Industrial Co., Ltd., (product name "Nocceler D")
Nocceler TP: Sodium dibutyldithiocarbamate, manufactured by Ouchi Shinko Chemical Industrial Co., Ltd., (product name "Nocceler TP")

Heteroaromatic Ring Compound (E)

Chemical formula 2: Heteroaromatic ring compound represented by chemical formula (2)

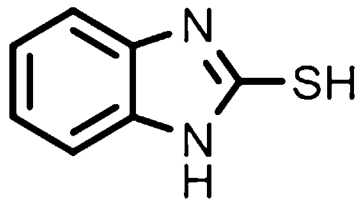

(2)

Chemical formula 3: Heteroaromatic ring compound represented by chemical formula (3)

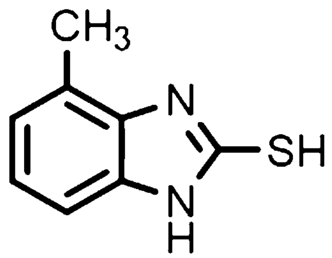

(3)

Chemical formula 4: Heteroaromatic ring compound represented by chemical formula (4)

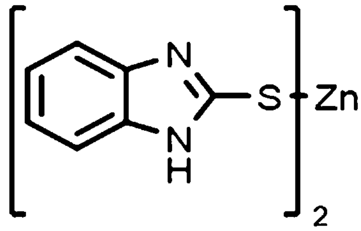

(4)

Antioxidant

Antioxidant: Butylated reaction product of p-cresol and dicyclopentadiene, manufactured by Ouchi Shinko Chemical Industrial Co., Ltd., (product name "Nocrac PBK")

<Evaluation of Dip-molded Body>

(Film Thickness)

The thickness (film thickness) of three locations in a central part of the dip-molded body film for evaluation was measured using a test piece thickness gauge (manufactured by Kobunshi Keiki Co., Ltd., product name "ASKER SDA-12"), and a minimum thickness was obtained as the thickness of the dip-molded body film for evaluation. The results are shown in Tables 1 to 3.

(Measurement of Tensile Properties)

Using the dip-molded body film for evaluation, a 100% elongation modulus and a tensile strength at break were measured in accordance with JIS K 6251. The results are shown in Tables 1 to 3.

(Toluene Swelling Degree)

The dip-molded body film for evaluation was cut into 10 mm × 15 mm to obtain a test piece. After measuring a mass W1 of the obtained test piece before immersion, the test piece was immersed in toluene for 20 hours in an environment of 23°C. After taking out the test piece after immersion from toluene and wiping off toluene on a film surface, a

mass W2 after immersion was measured. A toluene swelling degree was calculated from the measured mass W1 before immersion and mass W2 after immersion by the following Equation (1). The results are shown in Table 1.

$$\text{Equation (1): Toluene swelling degree} = 1 + (W2/W1 - 1)(P1/P2)(1/r)$$

P1: Specific gravity of chloroprene-based polymer component in chloroprene-based polymer latex (A)
P2: Specific gravity of toluene
r: Mass fraction of chloroprene-based polymer component in dip-molded body

Calculation was performed with P1 as 1.23 and P2 as 0.867. The chloroprene-based polymer component in the chloroprene-based polymer latex (A) can be obtained by mixing the chloroprene-based polymer latex (A) according to Examples and Comparative Examples with methanol to precipitate the chloroprene-based polymer component, followed by filtration and drying. The specific gravity of the chloroprene-based polymer component in the chloroprene-based polymer latex (A) obtained by filtration and drying was calculated by measuring the density of the chloroprene-based polymer obtained by the above method at room temperature (20°C).

The toluene swelling degree of Example 1 was 9.2, and the toluene swelling degree of Example 14 was 13.8.

3 g of the above-mentioned dip-molded body film for evaluation was cut into 2 mm squares to obtain a test piece. After putting this test piece into an eggplant-shaped flask equipped with a condenser, gas chromatography was performed under the following conditions using an extract obtained by extracting with an ethanol and toluene azeotropic mixture (ETA solution) defined by JIS K 6229 and performing hydrochloric acid treatment. From the measurement results of gas chromatography, a total peak area RA of dehydroabietic acid, pimaric acid, isopimaric acid, dihydroabietic acid, and salts thereof was determined. In addition, a total peak area RB of abietic acid, neoabietic acid, palustric acid, levopimaric acid, and salts thereof was determined, and a ratio RB/RA ((conjugated resin acid component b)/(non-conjugated resin acid component a)) of the total peak area RB of abietic acid, neoabietic acid, palustric acid, levopimaric acid, and salts thereof to the total peak area RA of dehydroabietic acid, pimaric acid, isopimaric acid, dihydroabietic acid, and salts thereof in the dip-molded body film for evaluation was calculated.

[Gas Chromatography Measurement Conditions]

Column used: FFAP 0.32 mmφ (diameter) × 25 m (film thickness 0.3 μm)
Detector: FID
Column temperature: 200° C (hold for 90 min) → 250° C
Heating rate: 10°C/min
Inlet temperature: 270°C
Detector temperature: 270°C
Injection volume: 2 μL

[Table 1]

| Table 1 | | | | Example 1 | Example 2 | Example 3 | Example 4 | Example 5 | Example 6 | Example 7 | Example 8 |
|---|---|---|---|---|---|---|---|---|---|---|---|
| | Sample name | | - | A-1 | A-1 | A-1 | A-1 | A-1 | A-1 | A-1 | A-1 |
| Chloroprene-based polymer latex (A) | Toluene-insoluble content | | % by mass | 69.1 | 69.1 | 69.1 | 69.1 | 69.1 | 69.1 | 69.1 | 69.1 |
| | Peak with weight average molecular weight of 5,000 to 80,000 | | - | 18456 | 18456 | 18456 | 18456 | 18456 | 18456 | 18456 | 18456 |
| Chloroprene-based polymer latex composition | Formulation | Solid content of chloroprene-based polymer latex (A) | parts by mass | 100 | 100 | 100 | 100 | 100 | 100 | 100 | 100 |
| | | Metal oxide (B) Type 2 zinc oxide ZnO | parts by mass | 2.0 | 2.0 | 2.0 | 4.9 | 0.6 | 2.0 | 2.0 | 2.0 |
| | | Vulcanization accelerator (C) Nocceler BZ | parts by mass | 0.5 | - | - | 0.5 | 0.5 | 0.5 | 0.5 | 0.5 |
| | | Vulcanization accelerator (C) Nocceler C | parts by mass | - | 0.5 | - | - | - | - | - | - |
| | | Vulcanization accelerator (C) Nocceler TET | parts by mass | - | - | 0.5 | - | - | - | - | - |
| | | Vulcanization accelerator (C) Nocceler D | parts by mass | - | 0.25 | - | - | - | - | - | - |
| | | Vulcanization accelerator (C) Nocceler TP | parts by mass | - | - | 0.25 | - | - | - | - | - |
| | | Sulfur (D) | parts by mass | 0.25 | 0.25 | 0.125 | 0.25 | 0.25 | 0.25 | 0.25 | 0.25 |
| | | Heteroaromatic ring compound (E) | type | Chemical formula 2 | Chemical formula 2 | Chemical formula 2 | Chemical formula 2 | Chemical formula 2 | Chemical formula 3 | Chemical formula 4 | Chemical formula 2 |
| | | Heteroaromatic ring compound (E) | parts by mass | 1 | 1 | 1 | 1 | 1 | 1 | 1 | 4.8 |
| | | Antioxidant | parts by mass | 2 | 2 | 2 | 2 | 2 | 2 | 2 | 2 |

(continued)

| Table 1 | | | Example | | | | | | | |
|---|---|---|---|---|---|---|---|---|---|---|
| | | | 1 | 2 | 3 | 4 | 5 | 6 | 7 | 8 |
| Properties of dip-molded body | RB/RA | - | 0.29 | 0.28 | 0.30 | 0.29 | 0.30 | 0.28 | 0.29 | 0.29 |
| | Film thickness | mm | 0.19 | 0.21 | 0.20 | 0.19 | 0.22 | 0.21 | 0.20 | 0.20 |
| | 100% elongation modulus | MPa | 0.55 | 0.57 | 0.56 | 0.59 | 0.55 | 0.62 | 0.60 | 0.60 |
| | Tensile strength at break | MPa | 22.4 | 22.3 | 22.0 | 21.9 | 20.3 | 21.2 | 20.4 | 20.8 |

[Table 2]

| Table 2 | | | | | Example | | | | | | |
|---|---|---|---|---|---|---|---|---|---|---|---|
| | | | | | 9 | 10 | 11 | 12 | 13 | 14 | 15 |
| Chloroprene-based polymer latex (A) | Sample name | | | - | A-1 | A-1 | A-1 | A-1 | A-13 | A-14 | A-15 |
| | Toluene-insoluble content | | | % by mass | 69.1 | 69.1 | 69.1 | 69.1 | 83.8 | 67.6 | 69.1 |
| | Peak with weight average molecular weight of 5,000 to 80,000 | | | - | 18456 | 18456 | 18456 | 18456 | None | 18456 | 18666 |
| Chloroprene-based polymer latex composition | Formulation | Solid content of chloroprene-based polymer latex (A) | | parts by mass | 100 | 100 | 100 | 100 | 100 | 100 | 100 |
| | | Metal oxide (B) | Type 2 zinc oxide ZnO | parts by mass | 2.0 | 2.0 | 2.0 | 2.0 | 2.0 | 2.0 | 2.0 |
| | | Vulcanization accelerator (C) | Nocceler BZ | parts by mass | 0.5 | 0.5 | 0.5 | 0.5 | 0.5 | 0.5 | 0.5 |
| | | | Nocceler C | parts by mass | - | - | - | - | - | - | - |
| | | | Nocceler TET | parts by mass | - | - | - | - | - | - | - |
| | | | Nocceler D | parts by mass | - | - | - | - | - | - | - |
| | | | Nocceler TP | parts by mass | - | - | - | - | - | - | - |
| | | Sulfur (D) | | parts by mass | 0.25 | 0.35 | 0.05 | 0.25 | 0.25 | 0.25 | 0.25 |
| | | Heteroaromatic ring compound (E) | | type | Chemical formula 2 | Chemical formula 2 | Chemical formula 2 | Chemical formula 2 | Chemical formula 2 | Chemical formula 2 | Chemical formula 2 |
| | | | | parts by mass | 0.4 | 1 | 1 | 1 | 1 | 1 | 1 |
| | | Antioxidant | | parts by mass | 2 | 2 | 2 | 0.3 | 2 | 2 | 2 |

(continued)

| Table 2 | | | Example | | | | | | |
|---|---|---|---|---|---|---|---|---|---|
| | | | 9 | 10 | 11 | 12 | 13 | 14 | 15 |
| Properties of dip-molded body | RB/RA | - | 0.30 | 0.27 | 0.29 | 0.27 | 0.28 | 0.29 | 0.00 |
| | Film thickness | mm | 0.21 | 0.18 | 0.19 | 0.21 | 0.19 | 0.21 | 0.20 |
| | 100% elongation modulus | MPa | 0.56 | 0.60 | 0.55 | 0.58 | 0.64 | 0.57 | 0.58 |
| | Tensile strength at break | MPa | 20.0 | 23.1 | 19.6 | 19.5 | 21.1 | 19.0 | 19.3 |

[Table 3]

| Table 3 | | | | | Comparative Example | | | | | | | |
|---|---|---|---|---|---|---|---|---|---|---|---|---|
| | | | | | 1 | 2 | 3 | 4 | 5 | 6 | 7 | 8 |
| Chloroprene-based polymer latex (A) | Sample name | | | - | A-1 | A-17 | A-1 | A-1 | A-1 | A-1 | A-1 | A-23 |
| | Toluene-insoluble content | | | % by mass | 69.1 | 67.0 | 69.1 | 69.1 | 69.1 | 69.1 | 69.1 | 45.6 |
| | Peak with weight average molecular weight of 5,000 to 80,000 | | | - | 18456 | 20200 | 18456 | 18456 | 18456 | 18456 | 18456 | 18456 |
| Chloroprene-based polymer latex composition | Formulation | Solid content of chloroprene-based polymer latex (A) | | parts by mass | 100 | 100 | 100 | 100 | 100 | 100 | 100 | 100 |
| | | Metal oxide (B) | Type 2 zinc oxide ZnO | parts by mass | 2.0 | 2.0 | 2.0 | 2.0 | 2.0 | 18.0 | - | 2.0 |
| | | Vulcanization accelerator (C) | Nocceler BZ | parts by mass | 2.0 | - | - | 0.5 | 0.5 | 0.5 | 0.5 | 0.5 |
| | | | Nocceler C | parts by mass | - | - | - | - | - | - | - | - |
| | | | Nocceler TET | parts by mass | - | - | - | - | - | - | - | - |
| | | | Nocceler D | parts by mass | - | - | - | - | - | - | - | - |
| | | | Nocceler TP | parts by mass | - | - | - | - | - | - | - | - |
| | | Sulfur (D) | | parts by mass | 1.00 | - | - | 0.25 | 0.25 | 0.25 | 0.25 | 0.25 |
| | | Heteroaromatic ring compound (E) | | type | Chemical formula 2 | Chemical formula 2 | Chemical formula 2 | Chemical formula 2 | - | Chemical formula 2 | Chemical formula 2 | Chemical formula 2 |
| | | | | parts by mass | 1 | 1 | 1 | 12 | - | 1 | 1 | 1 |
| | | Antioxidant | | parts by mass | 2 | 2 | 2 | 2 | 2 | 2 | 2 | 2 |

(continued)

| Table 3 | | | Comparative Example | | | | | | | |
|---|---|---|---|---|---|---|---|---|---|---|
| | | | 1 | 2 | 3 | 4 | 5 | 6 | 7 | 8 |
| Properties of dip-molded body | RB/RA | - | 0.30 | 0.28 | 0.29 | 0.27 | 0.29 | 0.30 | 0.29 | 0.27 |
| | Film thickness | mm | 0.20 | 0.20 | 0.19 | Not producible | 0.20 | 0.22 | 0.19 | 0.19 |
| | 100% elongation modulus | MPa | 0.75 | 0.61 | 0.57 | | 0.59 | 0.72 | 0.52 | 0.59 |
| | Tensile strength at break | MPa | 20.1 | 14.6 | 18.8 | | 14.6 | 19.0 | 15.7 | 15.4 |

## Claims

**1.** A chloroprene-based polymer latex composition comprising:

a chloroprene-based polymer latex (A),
a metal oxide (B),
a vulcanization accelerator (C),
sulfur (D), and
a heteroaromatic ring compound (E), wherein:

a toluene-insoluble content of a chloroprene-based polymer rubber obtained by freeze-drying the chloroprene-based polymer latex (A) is 50 to 95% by mass;
the chloroprene-based polymer latex composition comprises 0.3 to 15.0 parts by mass of the metal oxide (B), 0.02 to 1.50 parts by mass of the vulcanization accelerator (C), 0.01 to 0.75 parts by mass of the sulfur (D), and
0.1 to 10.0 parts by mass of the heteroaromatic ring compound (E), with respect to 100 parts by mass of a solid content of the chloroprene-based polymer latex (A);
the vulcanization accelerator (C) comprises at least one selected from a group consisting of a thiuram-based vulcanization accelerator, a dithiocarbamate-based vulcanization accelerator, a thiourea-based vulcanization accelerator, a guanidine-based vulcanization accelerator, a xanthate-based vulcanization accelerator, and a thiazole-based vulcanization accelerator; and
the heteroaromatic ring compound (E) is represented by general formula (1),

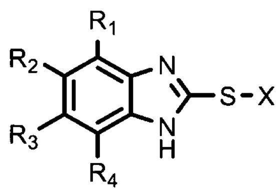

(1)

wherein, in the general formula (1), X represents a hydrogen atom or a metal atom, and $R_1$ to $R_4$ each independently represent a hydrogen atom, an optionally substituted alkyl group, an optionally substituted ether group, a nitro group, an amino group, or a carboxyl group, and $R_1$ to $R_4$ may be the same or different from each other.

**2.** The chloroprene-based polymer latex composition of Claim 1, comprising 0.5 to 10.0 parts by mass of an antioxidant with respect to 100 parts by mass of the solid content of the chloroprene-based polymer latex (A).

**3.** The chloroprene-based polymer latex composition of Claim 1 or Claim 2, wherein a molecular weight distribution obtained by gel permeation chromatography measurement of a tetrahydrofuran-soluble content in the chloroprene-based polymer latex (A) has a peak with a weight average molecular weight of 5,000 to 80,000.

**4.** The chloroprene-based polymer latex composition of Claim 1 or Claim 2, wherein a ratio RB/RA is 0.10 or more, wherein the ratio RB/RA is a ratio of a total peak area RB of abietic acid, neoabietic acid, palustric acid, levopimaric acid, and salts thereof to a total peak area RA of dehydroabietic acid, pimaric acid, isopimaric acid, dihydroabietic acid, and salts thereof, obtained from a gas chromatography analysis of an extract extracted with an ethanol and toluene azeotropic mixture defined by JIS K 6229 from a dip-molded body obtained by molding the chloroprene-based polymer latex composition by a dip coagulation method and heat-drying at 140°C for 60 minutes.

**5.** The chloroprene-based polymer latex composition of Claim 1 or Claim 2, wherein a dip-molded body, obtained by molding the chloroprene-based polymer latex composition by a dip coagulation method and heat-drying at 140°C for 60 minutes, has a 100% elongation modulus of 0.65 MPa or less, measured based on JIS K 6251, and
a tensile strength at break of 19.0 MPa or more, measured based on JIS K 6251.

**6.** A dip-molded body of the chloroprene-based polymer latex composition of Claim 1 or Claim 2.

**7.** The dip-molded body of Claim 6, wherein the dip-molded body is a glove for industrial or general household use, a medical glove, a balloon, a catheter, or a boot.

**INTERNATIONAL SEARCH REPORT**

International application No. **PCT/JP2024/005234**

**A. CLASSIFICATION OF SUBJECT MATTER**

***C08L 11/02***(2006.01)i; ***A41D 19/00***(2006.01)i; ***C08K 3/06***(2006.01)i; ***C08K 3/22***(2006.01)i; ***C08K 5/378***(2006.01)i
FI: C08L11/02; C08K3/22; C08K3/06; C08K5/378; A41D19/00 A

According to International Patent Classification (IPC) or to both national classification and IPC

**B. FIELDS SEARCHED**

Minimum documentation searched (classification system followed by classification symbols)

C08L11/02; A41D19/00; C08K3/06; C08K3/22; C08K5/378

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| A | CN 104911948 A (ZHEJIANG SCI-TECH UNIVERSITY) 16 September 2015 (2015-09-16) | 1-7 |
| A | CN 105295145 A (ANHUI JINGHONG SEALING ELEMENT TECHNOLOGY CO., LTD.) 03 February 2016 (2016-02-03) | 1-7 |
| A | CN 109206695 A (DEZHOU HELIJIA RUBBER & PLASTIC PRODUCTS CO., LTD.) 15 January 2019 (2019-01-15) | 1-7 |
| A | JP 2013-171711 A (SWCC SHOWA CABLE SYSTEMS CO., LTD.) 02 September 2013 (2013-09-02) | 1-7 |
| A | JP 2019-026681 A (DENKA CO., LTD.) 21 February 2019 (2019-02-21) | 1-7 |
| A | JP 2011-122141 A (SHOWA DENKO K.K.) 23 June 2011 (2011-06-23) | 1-7 |
| A | JP 2014-114342 A (DENKI KAGAKU KOGYO KABUSHIKI KAISHA) 26 June 2014 (2014-06-26) | 1-7 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **08 April 2024** | **23 April 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | <br>Telephone No. |

Form PCT/ISA/210 (second sheet) (July 2022)

INTERNATIONAL SEARCH REPORT

International application No.
PCT/JP2024/005234

C. DOCUMENTS CONSIDERED TO BE RELEVANT

| Category* | Citation of document, with indication, where appropriate, of the relevant passages | Relevant to claim No. |
|---|---|---|
| A | WO 2016/133193 A1 (DENKA CO., LTD.) 25 August 2016 (2016-08-25) | 1-7 |
| A | WO 2020/189516 A1 (DENKA CO., LTD.) 24 September 2020 (2020-09-24) | 1-7 |
| A | WO 2020/230746 A1 (DENKA CO., LTD.) 19 November 2020 (2020-11-19) | 1-7 |

Form PCT/ISA/210 (second sheet) (July 2022)

**INTERNATIONAL SEARCH REPORT**
**Information on patent family members**

International application No.
**PCT/JP2024/005234**

| Patent document cited in search report | Publication date (day/month/year) | Patent family member(s) | Publication date (day/month/year) |
|---|---|---|---|
| CN 104911948 A | 16 September 2015 | (Family: none) | |
| CN 105295145 A | 03 February 2016 | (Family: none) | |
| CN 109206695 A | 15 January 2019 | (Family: none) | |
| JP 2013-171711 A | 02 September 2013 | (Family: none) | |
| JP 2019-026681 A | 21 February 2019 | (Family: none) | |
| JP 2011-122141 A | 23 June 2011 | (Family: none) | |
| JP 2014-114342 A | 26 June 2014 | (Family: none) | |
| WO 2016/133193 A1 | 25 August 2016 | (Family: none) | |
| WO 2020/189516 A1 | 24 September 2020 | EP 3889186 A1<br>CN 113166281 A | |
| WO 2020/230746 A1 | 19 November 2020 | US 2022/0213235 A1<br>EP 3971218 A1<br>CN 113661181 A | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP HEI7292165 A
- JP 2014114342 A
- WO 2019009038 A
- JP 2019143002 A